# EUROPEAN PATENT APPLICATION

(11) **EP 4 703 471 A1**
(43) Date of publication of application: **04.03.2026**
(21) Application number: 24797119.5
(22) Date of filing: 25.04.2024
(51) Int. Cl.: C12N 15/09, C07K 14/11, C07K 19/00, C12N 7/01, C12N 9/12, C12N 15/44, C12N 15/54, C12N 15/62, C12N 15/86, C07K 16/00

(54) **METHOD FOR CONTROLLING TRANSCRIPTION AND/OR REPLICATION OF VRNA BY INFLUENZA VIRUS RDRP, AND USE OF SAME**

(30) Priority: 25.04.2023 JP 2023071781
(71) Applicant: Shinshu University, Matsumoto-shi, Nagano 390-8621 (JP)
(72) Inventor: OGASAWARA, Shinzi, Matsumoto-shi, Nagano 390-8621 (JP)
(74) Representative: Müller-Boré & Partner Patentanwälte PartG mbB
(86) International application number: PCT/JP2024/016232
(87) International publication number: WO 2024/225374

(57) **Abstract**

The method of the present disclosure is a method for controlling vRNA transcription and/or replication by influenza virus RdRp, comprising forming at least one selected from the group consisting of subunits constituting the RdRp and NP into a fusion protein with another protein.

## Description

### Technical Field

The present invention relates to a method for controlling vRNA transcription and/or replication by influenza virus RNA-dependent RNA polymerase (RdRp), and use of the same.

### Background Art

Influenza A virus (IAV) is one of the most common viruses that affect humans. Annual epidemics and frequent pandemics result in numerous deaths and significant economic losses. However, IAV has considerable potential for practical use as an oncolytic agent and vector due to the following advantages. First, IAV is a negative-strand RNA virus, whose viral RNA (vRNA) is not reverse transcribed and is therefore non-mutagenic. Second, unlike commonly used viral vectors such as adenovirus and adeno-associated virus, IAV can be used for repeated infections by selecting the appropriate hemagglutinin (HA) subtype. Third, there are many antiviral drugs approved or under development for IAV, and these antiviral drugs can be used for vector control. Finally, if a sequence of multiple consecutive basic amino acids is inserted into the cleavage site of HA, cleavage by proteases such as furin and PC6, which are present in all cells, can occur, causing systemic infection. Progress in the practical application of IAV has benefited from the development of plasmid-based reverse genetic techniques for the construction of recombinant IAV and the discovery of packaging signals that allow the incorporation of modified vRNA segments into virions.

A cause of the cytolytic activity of IAV is PB1-F2, one of its proteins, translocating to the mitochondria of infected cells, which can trigger apoptosis. Infection with IAV strongly induces the adaptive and innate immune systems, which also result in cytolytic/oncolytic effects. A key challenge in using IAV as an oncolytic agent is providing a means for IAV to target only tumor cells. Muster et al. reported that the primary function of IAV nonstructural protein 1(NS1) is to counteract or inhibit the antiviral response mediated by the double-stranded-RNA-activated protein kinase (PKR) (NPL 1), and developed NS1-deficient IAV (delNS1), which can grow only in melanoma resistant to type I interferon (NPLs 2 and 3). In order to further enhance the efficacy of delNS1 IAV, immunostimulatory activity was added by inserting the coding sequence for interleukin-15 into the viral genome (NPLs 4 and 5). This is also an example of IAV used as a vector. Another strategy for tumor cell-targeting is to replace the trypsin cleavage site of the HA protein of IAV with an elastase cleavage site, considering that elastase is highly expressed in pancreatic cancer cells (NPL 6). However, these oncolytic IAVs can identify only tumor cells under specific conditions.

Other applications of IAV involve its use as a vector, particularly as a replicable vector, to carry foreign genes for therapy (NPLs 7 and 8) and foreign genes for bioimaging (NPLs 9 and 10). The IAV genome contains eight negative-sense singlestranded vRNA segments for polymerase acidic protein (PA), polymerase basic protein 1 (PB1), polymerase basic protein 2 (PB2), nucleoprotein (NP), neuraminidase (NA), matrix protein (M), HA, and NS, and a foreign gene can be inserted into each segment (NPLs 11 to 14). Unlike vectors lacking replication ability (NPL 15) or virus-like particles lacking genomic material (NPL 16), the advantage of vectors with replication ability is that they can deliver foreign genes to the deep parts of organs.

### Citation List

### Non-patent Literature

NPL 1: Bergmann M, Garcia-Sastre A, Carnero E, Pehamberger H, Wolff K, Palese P, Muster T. Influenza virus NS1 protein counteracts PKR-mediated inhibition of replication. J Virol 2000; 74: 6203-6206.
NPL 2: Bergmann M, Romirer I, Sachet M, Fleischhacker R, Garcia-Sastre A, Palese P, Wolff K, Pehamberger H, Jakesz R, Muster T. A genetically engineered influenza A virus with ras-dependent oncolytic properties. Cancer Res 2001; 61: 8188-8193.
NPL 3: Muster T, Rajtarova J, Sachet M, Unger H, Fleischhacker R, Romirer I, Grassauer A, Url A, Garcia-Sastre A, Wolff K, Pehamberger H, Bergmann M. Interferon resistance promoters oncolysis by influenza virus NS1-deletion mutants. J Cancer2004; 110: 15-21.
NPL 4: van Rikxoort M, Michaelis M, Wolschek M, Muster T, Egorov A, Seipelt J, Doerr HW, Cinatl J Jr. Oncolytic effects of a novel influenza A virus expressing interleukin-15 from the NS reading frame. PLoS One 2012; 7: e36506.
NPL 5: Hock K, Laengle J, Kuznetsova I, Egorov A, Hegedus B, Dome B, Wekerle T, Sachet M, Bergmann M. Oncolytic influenza A virus expressing interleukin-15 decreases tumor growth in vivo. Surgery 2017; 161: 735-746.
NPL 6: Kuznetsova I, Arnold T, Aschacher T, Schwager C, Hegedus B, GarayT, Stukova M, Pisareva M, Pleschka S, Bergmann M, Egorov A. Targeting an oncolytic influenza A virus to tumor tissue by elastase. Mol Ther Oncolytics 2017; 7: 592-599.
NPL 7: Hamilton JR, Vijayakumar G, Palese P. A recombinant antibody-expressing influenza virus delays tumor growth in a mouse model. Cell Rep 2018; 22:1-7.
NPL 8: Wang J, Shu T, Deng W, Zheng Y, Liao M, Ye X, Han L, He P, Zheng X, Li T, Feng Y, Hu F, Li P, Sun C, Chen L, Li F, Feng L. Mucosal priming with a recombinant influenza A virus vectored vaccine elicits T-cell and antibody responses to HIV-1 inmice. J Virol 2021; 95: e00059-21.
NPL 9: Pan W, Dong Z, Li F, Meng W, Feng L, Niu X, Li C, Luo Q, Li Z, Sun C, Chen L. Visualizing influenza virus infection in living mice. Nat Commun 2013; 4: 2369.
NPL 10: Fukuyama S, Katsura H, Zhao D, Ozawa M, Ando T, Shoemaker JE, Ishikawa I, Yamada S, Neumann G, Watanabe S, Kitano H, Kawaoka Y. Multi-spectral fluorescent reporter influenza viruses (Color-flu) as powerful tools for in vivo studies. Nat Commun 2015; 6: 6600.
NPL 11: Li F, Feng L, Pan W, Cong Z, Li C, Sun C, Chen L. Generation of replication-competent influenza A viruses carrying reporter gene harbored in the neuraminidase segment. J Virol 2010; 84: 12075-12081.
NPL 12: Munier S, Rolland T, Diot C, Jacob Y, Naffakh N. Exploration of binary virus-host interactions using an infectious protein complementation assay. Mol Cell Proteomics 2013; 12: 2845-2855.
NPL 13: Breen M, Nogales A, Baker SF, Perex DR, Martinez-Sobrido L. Replication-competent influenza A and B viruses expressing a fluorescent dynamic timer protein for in vitro and in vivo studies. PLoS One 2016; 11: e0147723.
NPL 14: Froggatt HM, Burke KN, Chaparian RR, Miranda HA, Zhu X, Chambers BS, Heaton NS. Influenza A virus segments five and six can harbor artificial introns allowing expanded coding capacity. PLoS Pathog 2021; 17: e1009951.
NPL 15: Neumann G, Watanabe T, Kawaoka Y. Plasmid-driven formation of influenza virus-like particles. J Virol 2000; 74: 547-551.
NPL 16: Patel JM, Vartabedian VF, Kim MC, He S, Kang SM, Selvaraj P. Influenza virus-like particles engineered by protein transfer with tumor-associated antigens induces protective antitumor immunity. Biothchnol Bioeng 2015; 112: 1102-1110.

### Summary of Invention

### Technical Problem

However, vectors with replication ability can replicate in any cell in the body, which may pose safety issues. Therefore, regarding influenza virus vectors with replication ability, there is a strong demand for novel methods that can control their replication ability depending on the environment, for example.

An embodiment of the present invention has been made to solve the above problems, and an object thereof is to provide a novel method for controlling vRNA transcription and/or replication by influenza virus RdRp, and use thereof.

### Solution to Problem

The present invention is intended to solve the above problems and includes the following.
- A method for controlling vRNA transcription and/or replication by influenza virus RNA-dependent RNA polymerase (RdRp), comprising forming at least one selected from the group consisting of subunits constituting the RdRp and nucleoprotein (NP) into a fusion protein with another protein.
- Modified RdRp in which at least one of subunits constituting influenza virus RdRp is constructed as a fusion protein with another protein, and the other protein interacts with a target substance.
- A nucleic acid construct for controlling vRNA transcription and/or replication by RdRp, which expresses **NP** constructed as a fusion protein with another protein and contained in modified **NP,** in which **NP** of influenza virus is constructed as a fusion protein with another protein, and the other protein interacts with a target substance, or which expresses a modified viral gene corresponding to the **NP.**

### Advantageous Effects of Invention

According to one embodiment of the present invention, it is possible to provide a novel method for controlling vRNA transcription and/or replication by influenza virus RdRp, and use thereof.

### Brief Description of Drawings

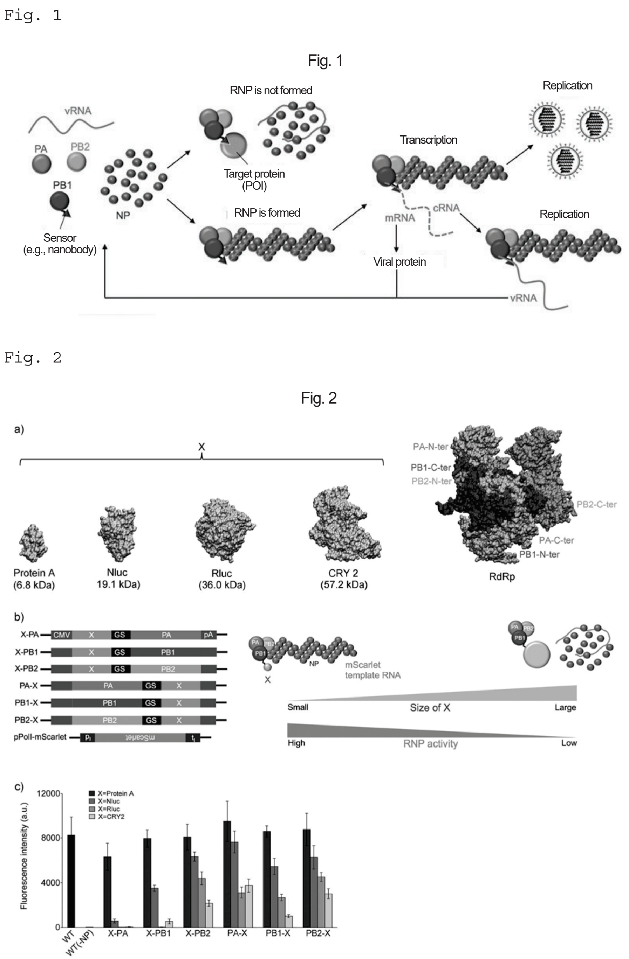

Fig. 1 is a schematic diagram of self-replicating IAV whose replication ability changes depending on the presence or absence of a target protein (POI). When RdRp binds to POI via a sensor protein (e.g., a nanobody) fused to the PA, PB1, or PB2 subunit, RdRp cannot form an RNP complex with NP and vRNA. Therefore, vRNA transcription and replication do not occur, and self-replicating IAV does not replicate. In contrast, RdRp performs vRNA transcription and replication through the formation of RNP complexes in the absence of POI, leading to viral replication.

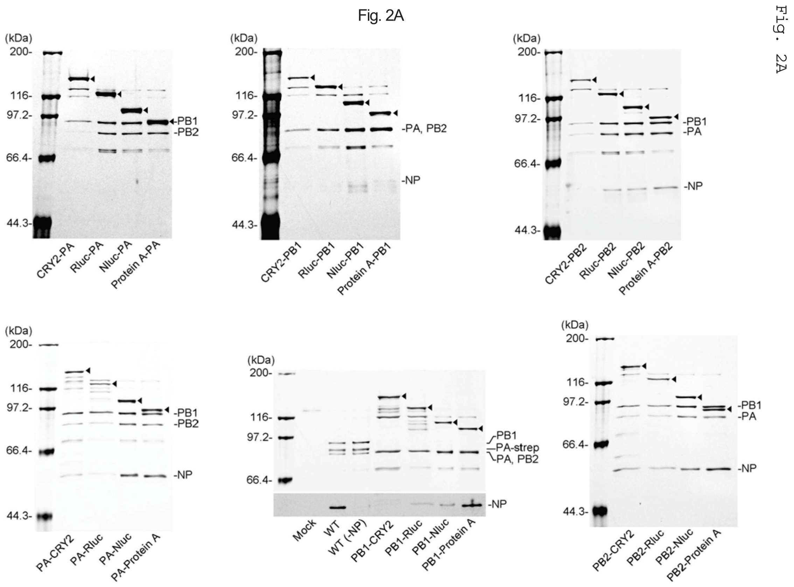

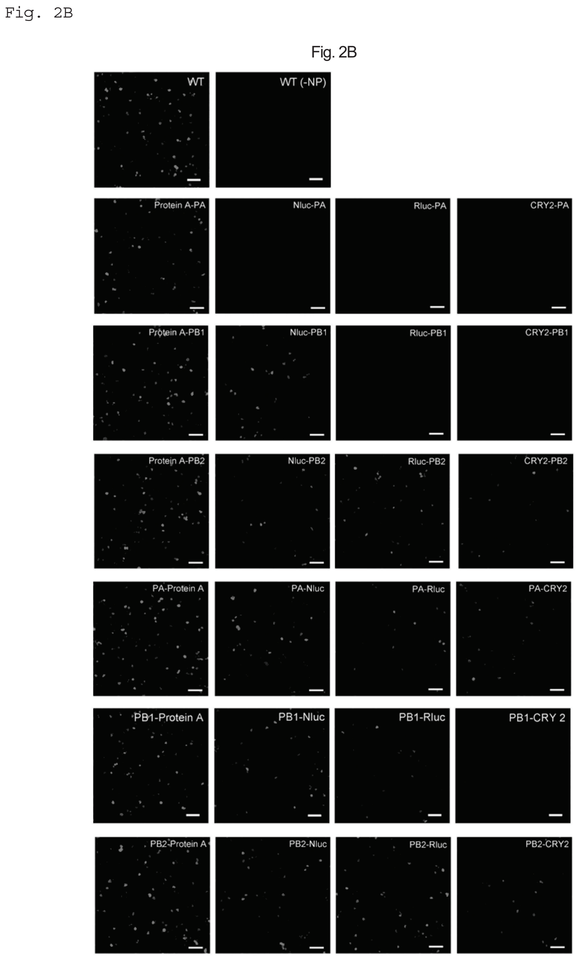

Fig. 2(a) is a diagram illustrating the space filling between proteins (X) fused to the PA, PB1, or PB2 subunit (B domein of Protein A; 1DEE, Nluc; 5IBO, Rluc; 2PSJ, CRY2; 7D0N) and RdRp (6RR7). Fig. 2(b) shows the structures of the plasmids used in the Examples. Protein (X) is fused to the N-terminus or C-terminus of the PA, PB1, or PB2 segment via a GS linker. CMV and pA represent the cytomegalovirus (CMV) promoter and BGH polyadenylation signal, respectively. The mScarlet-I sequence was inserted in a negative-sense orientation between the human RNA polymerase I promoter (p_{I}) sequence and the mouse RNA polymerase I terminator (t_{I}) sequence. The graph in Fig. 2(c) shows the mean ± standard deviation from three independent fluorescence intensity RNP activity assays (n = 3). The PA expression plasmid, PB1 expression plasmid, and PB2 expression plasmid were co-transfected with the NP expression plasmid and pPolI-mScarlet plasmid into HEK293T cells. After 48 hours, the fluorescence intensity of cell lysates was measured using a microplate reader.
Fig. 2A shows the results of RNP formation assays of X-PA, X-PB1, X-PB2, PA-X, PB1-X, and PB2-X. Recombinant RNPs purified using Strep-Tactin XT were analyzed by 7.5% SDS-PAGE. The arrowheads indicate the bands corresponding to each modified subunit.
Fig. 2B shows the fluorescence images of WT, WT (-NP), X-PA, X-PB1, X-PB2, PA-X, PB1-X, and PB2-X. The images were obtained at 48 hours post-transfection (hpt) using a confocal laser scanning microscope. The scale bar is 50 µm.

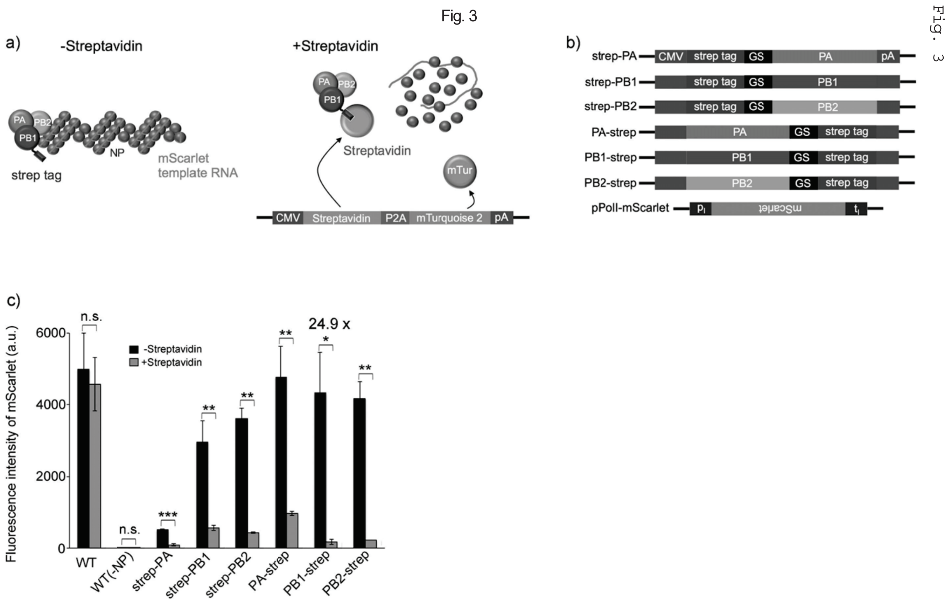

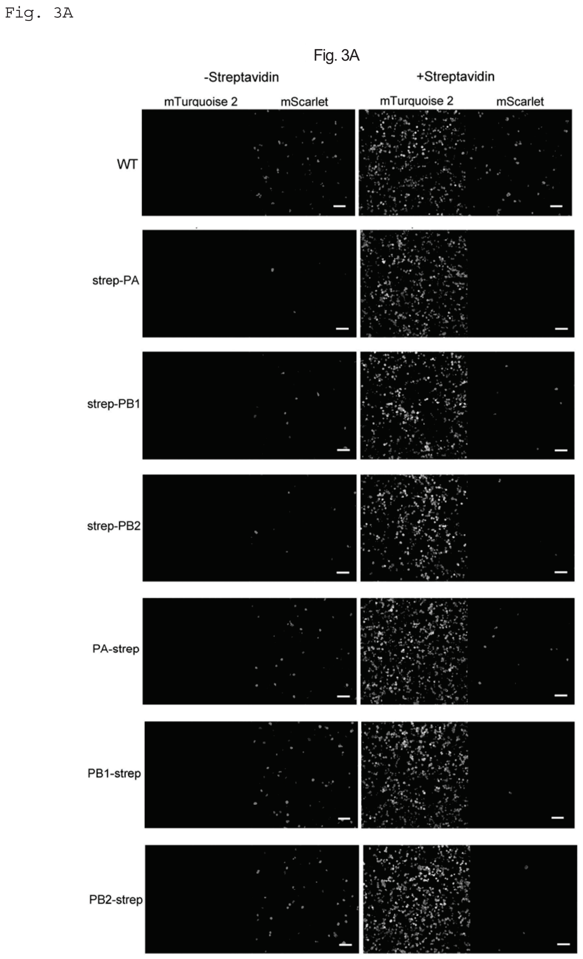

Fig. 3(a) is a schematic diagram showing RNP formation with and without streptavidin. Streptavidin and mTurquoise2 (mTur) were expressed separately from a single plasmid using P2A. Fig. 3(b) shows the structures of the plasmids used in the Examples. Strep-tag is fused to the N-terminus or C-terminus of PA, PB1, or PB2 via a GS linker. The graph in Fig. 3(c) shows the mean ± standard deviation from three independent fluorescence intensity RNP activity assays (n = 3). The PA expression plasmid, PB1 expression plasmid, and PB2 expression plasmid were co-transfected with the NP expression plasmid and pPolI-mScarlet plasmid into HEK293T cells in the presence or absence of a streptavidin expression plasmid. After 48 hours, the fluorescence intensity of cell lysates was measured using a microplate reader. Asterisks indicate p values from Welch's t-test (* p<0.05, ** p<0.01, *** p<0.001; n.s.: not significant difference).
Fig. 3A shows fluorescence images of WT and strep-fused subunits. The images were obtained at 48 hpt using a confocal laser scanning microscope. The scale bar is 50 µm.

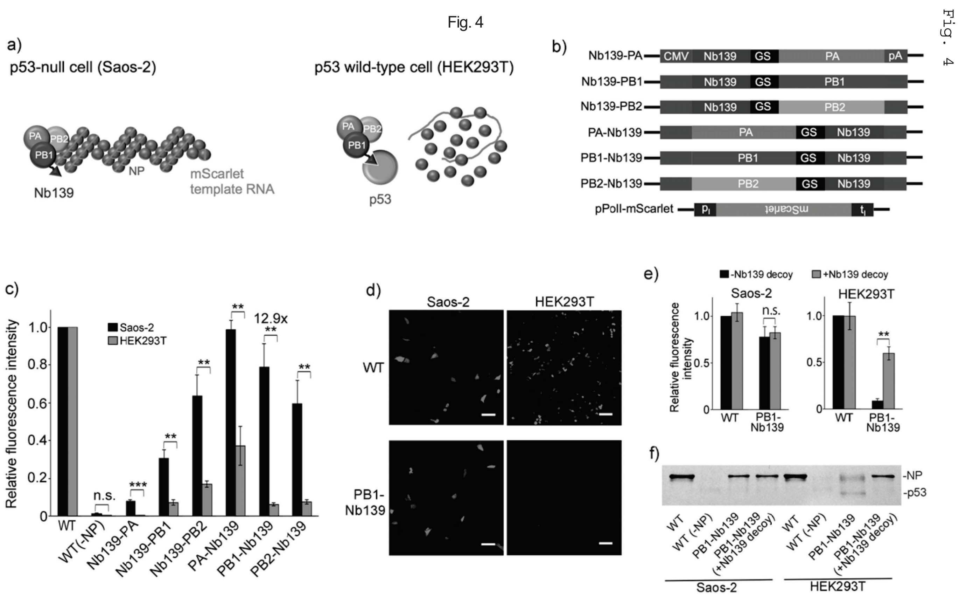

Fig. 4(a) is a schematic diagram illustrating RNP formation in the presence or absence of endogenous p53. Fig. 4(b) shows the structures of the plasmids used in the Examples. Nb139, which binds to the DNA-binding domain of p53, is fused to the N-terminus or C-terminus of PA, PB1, or PB2 via a GS linker. The graph in Fig. 4(c) shows the mean ± standard deviation from three independent fluorescence intensity RNP activity assays (n = 3). The PA expression plasmid, PB1 expression plasmid, and PB2 expression plasmid were co-transfected with the NP expression plasmid and pPolI-mScarlet plasmid into p53-expressing HEK293T or p53-deficient Saos-2 cells. After 48 hours, the fluorescence intensity of cell lysates was measured using a microplate reader. Fig. 4(d) shows fluorescence images of WT and PB1-Nb139. The images were obtained at 48 hpt using a confocal laser scanning microscope. The scale bar is 50 µm. Fig. 4(e) shows the activity of RNP in the presence of excess Nb139 as a decoy. The Nb139 expression plasmid was co-transfected with plasmids expressing RNP components. Asterisks indicate p values from Welch's t-test (* p<0.05, ** p<0.01, *** p<0.001; n.s.: not significant difference). Fig. 4(f) shows the results of a pull-down assay of endogenous p53 in Saos-2 cells and HEK293T cells expressing RNP containing PB1-Nb139. At 48 hpt, RNPs were purified from cell lysates using the interaction between the strep-tag fused to the C-terminus of the PA subunit and Strep-Tactin XT.

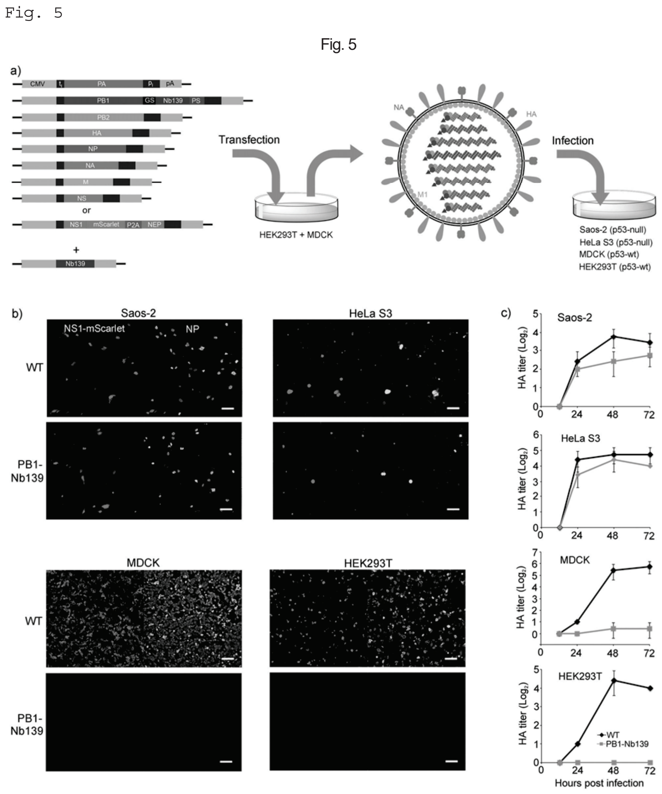

Fig. 5(a) is a schematic diagram showing the generation of self-replicating IAV using an eight-plasmid system. Each cDNA encoding the IAV gene flanked by the human RNA polymerase I promoter (p_{I}) and the mouse RNA polymerase I terminator (t_{I}) was inserted between the BGH polyadenylation signal (pA) and the CMV promoter (CMV). Because HEK293T and MDCK cells express wild-type p53, Nb139 expression plasmid was co-transfected with PA expression plasmid, PB1-Nb139 expression plasmid, PB2 expression plasmid, HA expression plasmid, NP expression plasmid, NA expression plasmid, M expression plasmid, and NS (or NS-mScarlet) expression plasmid to mask endogenous p53. In the NS-mScarlet expression plasmid, the NS segment is divided into NS1 and NEP ORFs by inserting the mScarlet-I ORF accompanied by a P2A site, thereby allowing the NEP protein and NS1-mScarlet-I protein to be produced separately. PS stands for packaging signal. Fig. 5(b) shows visualization of infection and replication of wild-type IAV or PB1-Nb139-containing IAV using red fluorescence from NS1-mScarlet-I and green fluorescence from NP stained with anti-NP MAb followed by FITC-conjugated anti-mouse IgG secondary antibody. MDCK cells (p53-wt), HEK293T cells (p53-wt), Saos-2 cells (p53-null), and HeLa S3 cells (p53-null) were infected with IAV at MOIs (multiplicities of infection) of 0.1, 1, 1, and 1, respectively. At 24 hours post-infection (24 hpi), fluorescent images were obtained using a confocal laser scanning microscope. The scale bar is 50 µm. Fig. 5(c) shows the changes over time in HA titers of wild-type IAV or PB1-Nb139-containing IAV. MDCK cells, HEK293T cells, Saos-2 cells, and HeLa S3 cells were infected with IAV at MOIs of 0.01, 0.1, 5, and 5, respectively. The supernatant of the culture medium in which the infected cells were cultured was sampled at 12, 24, 48, and 72 hours after infection, and assayed for HA. The graph shows the mean ± standard deviation from three independent HA titer assays (n = 3).

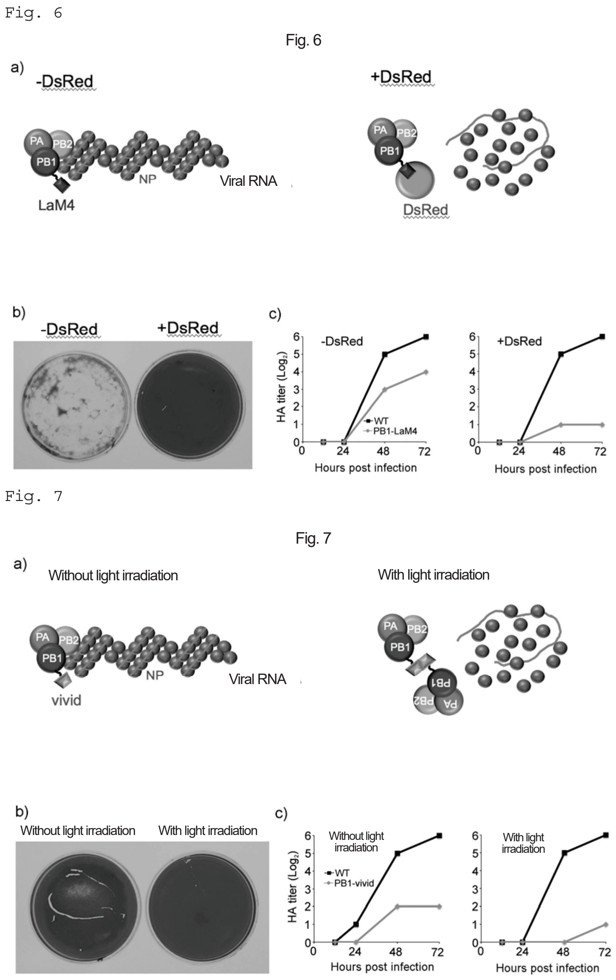

Fig. 6(a) is a schematic diagram illustrating RNP formation in the presence or absence of red fluorescent protein (DsRed). A nanobody (LaM4) that binds to DsRed is fused to the C-terminus of PB1 via a GS linker. Fig. 6(b) is an image showing cell degeneration (apoptosis) due to the replication of PB1-LaM4-containing IAV. MDCK cells and DsRed-expressing MDCK cells were infected with PB1-LaM4-containing IAV at an MOI of 0.1. At 48 hours post-infection (48 hpi), the cells were stained with crystal violet. The graph in Fig. 6(c) shows the changes over time in HA titers of wild-type IAV or PB1-LaM4-containing IAV. MDCK cells and DsRed-expressing MDCK cells were infected with wild-type IAV or PB1-LaM4-containing IAV at an MOI of 0.01. The supernatant of the culture medium in which the infected cells were cultured was sampled at 12, 24, 48, and 72 hours after infection, and HA assays were performed.
Fig. 7(a) is a schematic diagram illustrating RNP formation with and without light irradiation. Vivid, which forms a homodimer upon irradiation with 460 nm blue light, is fused to the C-terminus of PB1 via a GS linker. Fig. 7(b) is an image showing cell degeneration (apoptosis) due to the replication of PB1-vivid-containing IAV. MDCK cells were infected with PB1-vivid-containing IAV. At 48 hours post-infection (48 hpi), the cells were stained with crystal violet. The graph in Fig. 7(c) shows the changes over time in HA titers of wild-type IAV or PB1-vivid-containing IAV. MDCK cells were infected with wild-type IAV or PB1-vivid-containing IAV at the same titer. The supernatant of the culture medium in which the infected cells were cultured was sampled at 12, 24, 48, and 72 hours after infection, and HA assays were performed.

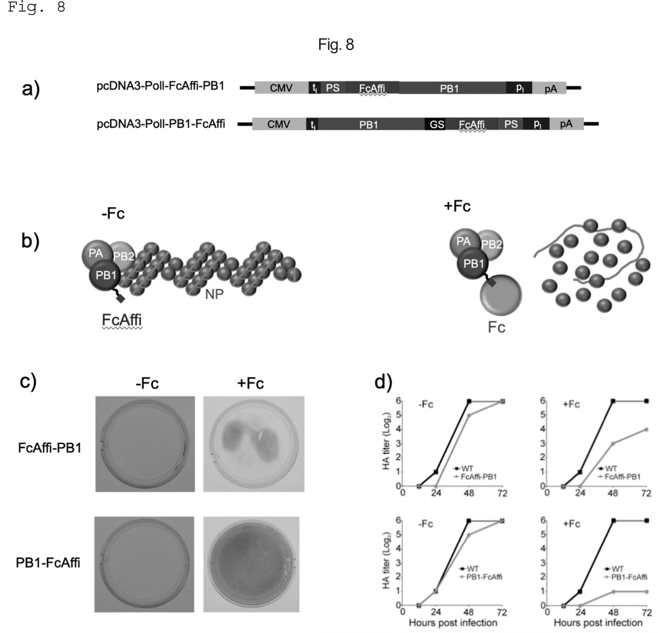

Fig. 8(a) shows the structures of the plasmids expressing PB1 protein and vRNA used to generate self-replicating IAV containing FcAffi-PB1 or PB1-FcAffi. The cDNA encoding the PB1 gene flanked by the human RNA polymerase I promoter (p_{I}) and the mouse RNA polymerase I terminator (t_{I}) was inserted between the BGH polyadenylation signal (pA) and the CMV promoter (CMV). Affibody (FcAffi) that binds to the Fc region of IgG is fused to the N-terminus of PB1 without a linker in the FcAffi-PB1 plasmid, and to the C-terminus of PB1 via a GS linker in the PB1-FcAffi plasmid. Fig. 8(b) is a schematic diagram illustrating RNP formation in the presence or absence of an IgG Fc region protein (Fc). Fig. 8(c) is an image showing cell degeneration (apoptosis) due to the replication of FcAffi-PB1- or PB1-FcAffi-containing IAV. MDCK cells not expressing Fc and MDCK cells expressing Fc were infected with FcAffi-PB1- or PB1-FcAffi-containing IAV at an MOI of 0.1. At 48 hours post-infection (48 hpi), the cells were stained with crystal violet. The graph in Fig. 8(d) shows the changes over time in HA titers of wild-type IAV or FcAffi-PB1- or PB1-FcAffi-containing IAV. MDCK cells not expressing Fc and MDCK cells expressing Fc were infected with wild-type IAV or FcAffi-PB1- or PB1-FcAffi-containing IAV at an MOI of 0.01. The supernatant of the culture medium in which the infected cells were cultured was sampled at 12, 24, 48, and 72 hours after infection, and HA assays were performed.

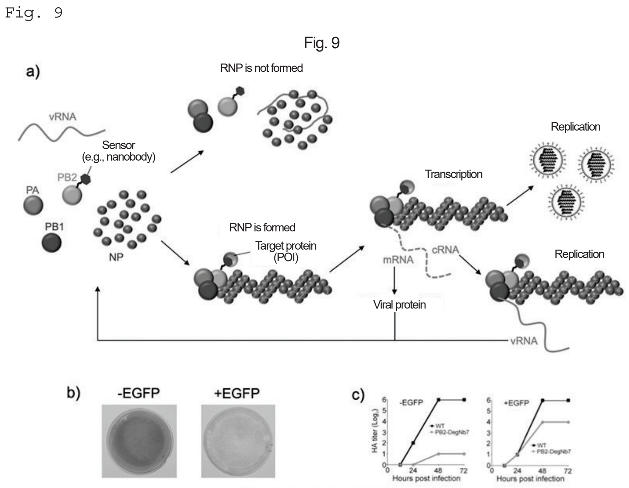

Fig. 9(a) is a schematic diagram of self-replicating IAV whose replication ability changes depending on the presence or absence of a target protein (POI). When RdRp binds to POI via a sensor protein (e.g., a nanobody) fused to the PA, PB1, or PB2 subunit, RdRp forms an RNP complex with NP and vRNA. Therefore, vRNA transcription and replication occur, and self-replicating IAV replicates. In contrast, RdRp does not form an RNP complex in the absence of POI, and vRNA transcription and replication do not occur; thus, the virus does not replicate. Fig. 9(b) is an image showing cell degeneration (apoptosis) due to the replication of IAV containing PB2-DegNb7, in which a nanobody (DegNb7) that specifically binds to EGFP was fused to the C-terminus of PB2. MDCK cells not expressing EGFP and MDCK cells expressing EGFP were infected with PB2-DegNb7-containing IAV at an MOI of 0.1. At 48 hours post-infection (48 hpi), the cells were stained with crystal violet. In the MDCK cells not expressing EGFP, the replication of PB2-DegNb7-containing IAV is inhibited, and only slight cell degeneration is observed. In contrast, in the MDCK cells expressing EGFP, PB2-DegNb7-containing IAV replicate efficiently, causing most cells to undergo apoptosis. The graph in Fig. 9(c) shows the changes over time in HA titers of wild-type IAV or PB2-DegNb7-containing IAV. MDCK cells not expressing EGFP and MDCK cells expressing EGFP were infected with wild-type IAV or PB2-DegNb7-containing IAV at an MOI of 0.01. The supernatant of the culture medium in which the infected cells were cultured was sampled at 12, 24, 48, and 72 hours after infection, and HA assays were performed.

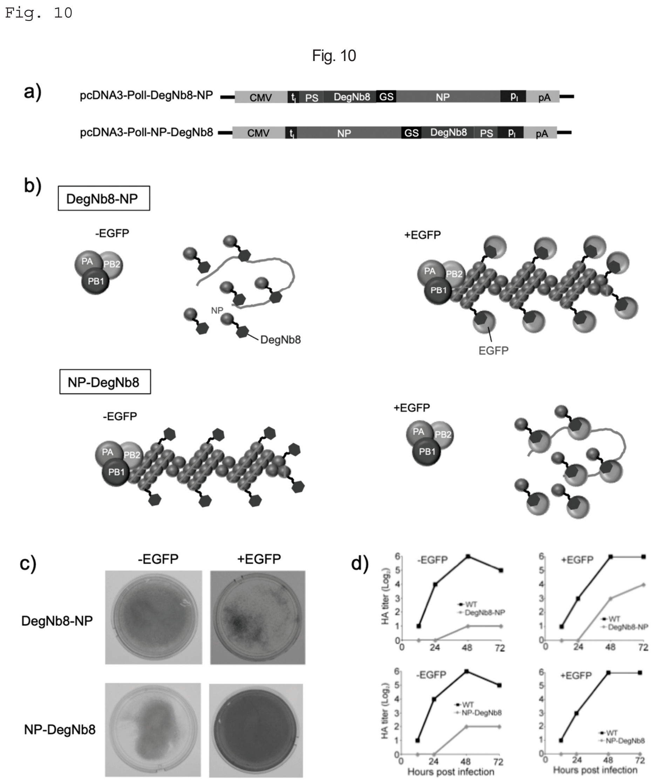

Fig. 10(a) shows the structures of the plasmids expressing NP protein and vRNA used to generate self-replicating IAV containing DegNb8-NP or NP-DegNb8, in which a nanobody (DegNb8) that specifically binds to EGFP is fused to the N-terminus or C-terminus of NP. The cDNA encoding the NP gene flanked by the human RNA polymerase I promoter (p_{I}) and the mouse RNA polymerase I terminator (t_{I}) was inserted between the BGH polyadenylation signal (pA) and the CMV promoter (CMV). DegNb8 is fused to the N-terminus or C-terminus of NP via a GS linker. Fig. 10(b) is a schematic diagram illustrating RNP formation in the presence or absence of EGFP. In DegNb8-NP-containing IAV, RNPs are formed in the presence of EGFP, and RNP formation is inhibited in the absence of EGFP. On the other hand, in NP-DegNb8-containing IAV, RNP formation is inhibited in the presence of EGFP, and RNPs are formed in the absence of EGFP. Fig. 10(c) is an image showing cell degeneration (apoptosis) due to the replication of DegNb8-NP- or NP-DegNb8-containing IAV. MDCK cells not expressing EGFP and MDCK cells expressing EGFP were infected with DegNb8-NP- or NP-DegNb8-containing IAV at an MOI of 1. At 48 hours post-infection (48 hpi), the cells were stained with crystal violet. The graph in Fig. 10(c) shows the changes over time in HA titers of wild-type IAV or DegNb8-NP- or NP-DegNb8-containing IAV. MDCK cells not expressing EGFP and MDCK cells expressing EGFP were infected with wild-type IAV or DegNb8-NP- or NP-DegNb8-containing IAV at an MOI of 0.1. The supernatant of the culture medium in which the infected cells were cultured was sampled at 12, 24, 48, and 72 hours after infection, and HA assays were performed.

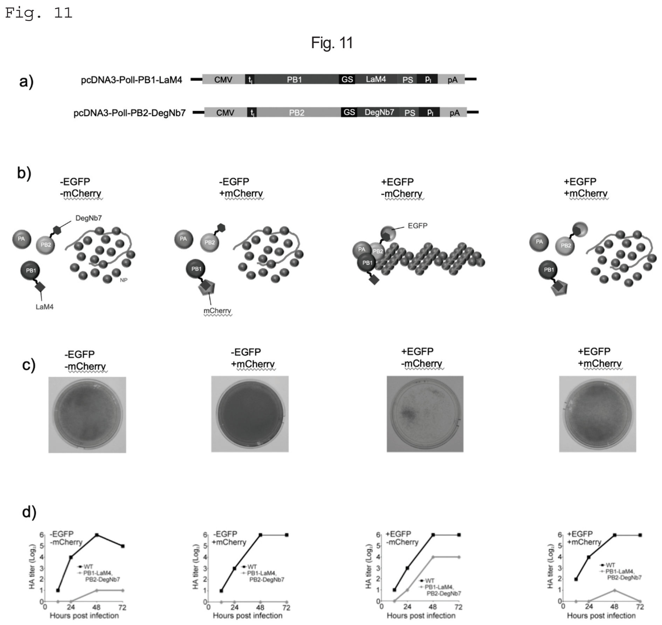

Fig. 11(a) shows the structures of the plasmids expressing PB1 and PB2 proteins and vRNA used to generate self-replicating IAV containing two types of nanobodies, LaM4 and DegNb7. The cDNA encoding the PB1 or PB2 gene flanked by the human RNA polymerase I promoter (p_{I}) and the mouse RNA polymerase I terminator (t_{I}) was inserted between the BGH polyadenylation signal (pA) and the CMV promoter (CMV). LaM4 is fused to the C-terminus of PB1, and DegNb7 is fused to the C-terminus of PB2, each via a GS linker. Fig. 11(b) is a schematic diagram illustrating RNP formation in the presence or absence of EGFP and mCherry. RNPs are formed only when EGFP alone is present. Fig. 11(c) is an image showing cell degeneration (apoptosis) due to the replication of IAV containing PB1-LaM4 and PB2-DegNb7. MDCK cells expressing neither EGFP nor mCherry, MDCK cells expressing EGFP, MDCK cells expressing mCherry, and MDCK cells expressing both EGFP and mCherry were infected with IAV containing PB1-LaM4 and PB2-DegNb7 at an MOI of 1. At 48 hours post-infection (48 hpi), the cells were stained with crystal violet. The graph in Fig. 11(d) shows the changes over time in HA titers of wild-type IAV or IAV containing PB1-LaM4 and PB2-DegNb7. MDCK cells expressing neither EGFP nor mCherry, MDCK cells expressing EGFP, MDCK cells expressing mCherry, and MDCK cells expressing both EGFP and mCherry were infected with wild-type IAV or IAV containing PB1-LaM4 and PB2-DegNb7 at an MOI of 0.1. The supernatant of the culture medium in which the infected cells were cultured was sampled at 12, 24, 48, and 72 hours after infection, and HA assays were performed.

### Description of Embodiments

### 1. Method for Controlling vRNA Transcription and/or Replication by RdRp

### Overview

The method according to the present embodiment is a method for controlling vRNA transcription and/or replication by influenza virus RNA-dependent RNA polymerase (hereinafter, "RdRp"), comprising forming at least one selected from the group consisting of subunits constituting the RdRp and nucleoprotein (NP) into a fusion protein with another protein.

### Influenza Virus

In the present embodiment, "influenza virus" refers to a virus belonging to the family *Orthomyxoviridae* and is classified as influenza virus. Specific examples include influenza A, B, C, and D viruses. From the viewpoint of application to humans, influenza A or B viruses may be preferred.

### Subunits Constituting RdRp

Influenza virus RdRp is a complex composed of three subunits: PB1, PB2, and PA. In the present embodiment, at least one of the subunits constituting RdRp is a fusion protein with another protein. Unless a specific subunit is mentioned, the subunit(s) constituting RdRp may refer to one, two, or all three of these subunits.

### NP

The NP of influenza virus is a protein that forms a ribonucleoprotein (RNP) complex together with RdRp and viral RNA (hereinafter referred to as "vRNA"). Multiple (e.g., 30 or more and 120 or less) NPs are present in an RNP complex.

Hereinafter, RdRp and NP may be collectively referred to as "the RNP-constituting protein."

### Control of vRNA Transcription and/or Replication

In an embodiment of the present invention, at least one of the subunits constituting RdRp is constructed as a fusion protein with another protein, resulting in modified RdRp. This allows vRNA transcription and/or replication to be controlled, for example, in an inhibitory direction, compared to the case with wild-type RdRp. In an embodiment of the present invention, the mechanism by which vRNA transcription and/or replication is controlled is considered to be that the presence of fusion proteins inhibits RNP formation.

Further, in an embodiment of the present invention, NP is constructed as a fusion protein with another protein, resulting in modified NP. Furthermore, in an embodiment of the present invention, at least one of the subunits constituting RdRp and NP are constructed as fusion proteins with other proteins, resulting in modified RdRp and NP.

In an embodiment of the present invention, the modified RNP-constituting protein can control vRNA transcription and/or replication in an inhibitory direction, compared to wild-type RNP-constituting protein. This is considered to be because the presence of the fusion protein inhibits RNP formation. In another embodiment of the present invention, the modified RNP-constituting protein can acquire transcription and replication activity when the other protein binds to a target substance. This is considered to be because the binding of the other protein to the target substance reduces the inhibition of RNP formation.

A preferred example of the other protein is a protein that interacts with a target substance. The interaction between the other protein and the target substance may cause changes in size, structure, etc. of the modified RdRp. As a result, the state of vRNA transcription and/or replication by the modified RdRp may change between a state in which the other protein and the target substance interact and a state in which they do not interact. In a more specific example, the state of vRNA transcription and/or replication by the modified RdRp may change depending on the amount of the target substance in the environment. In another more specific example, the state of vRNA transcription and/or replication by the modified RdRp may change between cells with different amounts of the target substance. This allows, for example, cells with different amounts of the target substance to be distinguished based on differences in the state of vRNA transcription and/or replication (this state can be visualized as necessary). When the amount of the target substance differs between normal cells and abnormal cells (e.g., diseased cells), this technique can be used to assist in pathological diagnosis. Furthermore, by taking advantage of the differences in the state of vRNA transcription and/or replication between cells with different amounts of the target substance, it is possible to induce or inhibit vRNA transcription and/or replication specifically in target cells. That is, the other protein functions as a sensor protein for the presence and amount of the target substance in the environment. The POI shown in the Examples is an example of the target substance.

Changes in size, structure, etc. due to the interaction between the other protein and the target substance can be broadly divided into two categories: 1) a case in which the size increases and the structure etc. also change due to the addition of the target substance, and 2) a case in which the other protein becomes smaller in size and also changes in structure etc.
- In case 1), the other protein is not particularly limited, but is preferably, for example, any one selected from the group consisting of a) a protein with specific binding affinity to a target substance, b) a light-responsive protein, c) an ion-responsive protein, d) a small molecule-binding protein, e) a nucleic acid-binding protein, and f) a temperature-responsive protein.

Examples of a) above include, but are not particularly limited to, antibody fragments with antigen-binding properties, such as nanobody and scFv; antibody-like proteins, such as DARPin, monobody, HLH peptide, Affibody, Anticalin, Knottin, Kunitz, Affimer, Nanofitin, Avimer, Centyrins, and affilin ADAPT; and proteins with specific binding affinity to target substances, such as strep-tag.

Examples of b) above include, but are not particularly limited to, proteins that form homo- or heteromultimers or dissociate multimers when irradiated with light of a specific wavelength, such as vivid, UVR8, RsLOV, PpSB1, YtvA, Dronpa, CRY2, PhyB, BphP1, cobalamin-binding domain, and PixD; or proteins whose structure changes when irradiated with light of a specific wavelength, such as AsLOV2, BcLOV4, AtLOV2, and PYP.

Examples of the ion-responsive protein c) above include, but are not particularly limited to, the BON-LysM system, the Mg ion-binding domain of CorA, the Mg ion-binding domain of HsCen3, the CaM-M13 system, the Atox1-WD4 system, and Amt1.

Examples of the small molecule-binbind protein d) above include, but are not particularly limited to, the cyclic nucleotide-binding domain of Epac1, the cyclic nucleotide-binding domain of PfPKG, the ε-subunit of ATP synthase, and sets of GTPases and their binding proteins (e.g., the Rac1-PAK1 system).

Examples of the nucleic acid-binding protein e) above include, but are not particularly limited to, HMGB1 and DHX36.

Examples of the temperature-responsive protein f) above include, but are not particularly limited to, elastin-like polypeptides (ELPs).
- In case 2), examples of the other protein include, but are not particularly limited to, proteins that are cleaved or degraded upon interaction with target substances. In an example, the other protein contains a cleavage site for a specific protease (target substance), such as elastase, furin, or PC5/6. In this case, the other protein becomes smaller in size and also changes in structure etc. due to the interaction with the protease.

The other protein interacts with the target substance within cells unless it is in an in vitro system. The type of target substance may be selected from the viewpoint of, for example, whether it is present specifically in target cells, or whether it is substantially absent in target cells but present in other cells.
- The interaction between the other protein and the target substance may reduce the hydrodynamic size, which may change the structure etc. of the RNP-constituting protein that forms a fusion protein with the other protein.
- The interaction between the other protein and the target substance may stabilize the structure of the other protein and/or the RNP-constituting protein that forms a fusion protein with the other protein.

### Subunit of RdRp as Fusion Protein

As described above, in an embodiment of the present invention, at least one of the subunits constituting RdRp is a fusion protein with another protein. The positional relationship between the subunit and the other protein in the fusion protein is not particularly limited as long as RdRp can be formed; however, from the viewpoint of not impairing the activity of the subunit and facilitating the interaction between the other protein and the target substance, it is preferable to place the other protein on the N-terminal or C-terminal side of the subunit. A spacer sequence may be present between the other protein and the subunit.

When the other protein is placed on the N-terminal side of the subunit, PA, PB1, and PB2, in this order, tend to be able to increase the size of the other protein itself or the total size of the other protein and the target substance.

When the other protein is placed on the C-terminal side of the subunit, PA tends to be able to increase the size of the other protein itself or the total size of the other protein and the target substance, compared to PB1 and PB2. In addition, when comparing PB1 and PB2, PB1 tends to inhibit the state of vRNA transcription and/or replication more inversely proportional to the size.

Regardless of the subunit, placing the other protein on the C-terminal side of the subunit tends to be able to increase the size of the other protein itself or the total size of the other protein and the target substance.

### NP as Fusion Protein

As described above, in an embodiment of the present invention, NP is a fusion protein with another protein. The positional relationship between NP and the other protein in the fusion protein is not particularly limited; however, from the viewpoint of not impairing the activity of NP and facilitating the interaction between the other protein and the target substance, it is preferable to place the other protein on the N-terminal or C-terminal side of NP. A spacer sequence may be present between the other protein and NP.

### Examples of Size of Other Protein

The size of the other protein constituting the fusion protein is not particularly limited as long as the intended purpose can be achieved; however, from the viewpoint that it is less likely to inhibit the formation of RdRp, the size may be preferably 100 kDa or less, and more preferably 90 kDa or less, 80 kDa or less, 70 kDa or less, 65 kDa or less, 60 kDa or less, 55 kDa or less, 50 kDa or less, 45 kDa or less, or 40 kDa or less. However, as will be explained in detail below, the preferred size may vary depending on the purpose and other factors.

1) Case in which the size increases and the structure etc. also change due to the addition of the target substance to the other protein:
   - Total size (A) of other protein and target substance: 10 kDa (molecular weight: 10,000) or more, 20 kDa or more, 30 kDa or more, 35 kDa or more, 40 kDa or more, 50 kDa or more, 55 kDa or more, 60 kDa or more, 65 kDa or more, 70 kDa or more, 80 kDa or more, 90 kDa or more, or 100 kDa or more (molecular weight: 100,000).
   - Size (B) of other protein: 35 kDa or less, 30 kDa or less, 25 kDa or less, 20 kDa or less, 15 kDa or less, 10 kDa or less, or 7 kDa or less. The lower limit is not particularly limited, but is, for example, 0.5 kDa or more, 1 kDa or more, 2 kDa or more, 3 kDa or more, 4 kDa or more, or 5 kDa or more.
   - However, it is a prerequisite that the relationship of size (A) > size (B) is satisfied. In this case, when the target substance is attached to the other protein, vRNA transcription and/or replication is more strongly inhibited, either completely or partially.
2) Case in which the other protein becomes smaller in size and also changes in structure etc. due to the interaction with the target substance
   - Size (A) of other protein: 10 kDa or more, 20 kDa or more, 30 kDa or more, 35 kDa or more, 40 kDa or more, 50 kDa or more, 55 kDa or more, 60 kDa or more, 65 kDa or more, 70 kDa or more, 80 kDa or more, 90 kDa or more, or 100 kDa or more.
   - Size (B) of other protein after interaction: 35 kDa or less, 30 kDa or less, 25 kDa or less, 20 kDa or less, 15 kDa or less, 10 kDa or less, or 7 kDa or less. The lower limit is not particularly limited, but is, for example, 0.5 kDa or more, 1 kDa or more, 2 kDa or more, 3 kDa or more, 4 kDa or more, or 5 kDa or more.
   - However, it is a prerequisite that the relationship of size (A) > size (B) is satisfied. In this case, when the target substance does not interact with the other protein, vRNA transcription and/or replication is more strongly inhibited, either completely or partially.
   - Alternatively, it is a prerequisite that the interaction with the target substance satisfies the relationship of (hydrodynamic diameter of other protein before interaction) > (hydrodynamic diameter of target substance bound to other protein after interaction).

The above specific examples and preferred examples of the other protein also apply to the case in which NP is constructed as a fusion protein with another protein.

### Environments in Which Control of vRNA Transcription and/or Replication by RdRp Occurs

Examples of environments in which control of vRNA transcription and/or replication by RdRp occurs include a cellfree environment and an intracellular environment. In the case of an intracellular environment, the cells are not particularly limited as long as they are host-derived cells infected with influenza virus. Hosts infected with influenza virus are not particularly limited, but examples include birds, humans, and non-human animals. More specifically, preferred examples include birds and mammals. Examples of mammals include laboratory animals, such as mice, rats, rabbits, guinea pigs, and primates other than humans; pet animals, such as dogs and cats; livestock, such as pigs, cows, goats, sheep, and horses; and humans. The intracellular environment may be an in vitro environment at the cellular or tissue level, or an in vivo intracellular environment.

In terms of easier control of vRNA transcription and/or replication, it is preferable to combine at least one of the subunits constituting RdRp with another protein to form a fusion protein.

### 2. Modified RNP-Constituting Protein

In the modified RdRp according to an embodiment of the present invention, at least one of the subunits constituting influenza virus RdRp is constructed as a fusion protein with another protein, and the other protein interacts with a target substance. Furthermore, in the RNP-constituting protein according to an embodiment of the present invention, at least one selected from the group consisting of the subunits constituting influenza virus RdRp and NP is constructed as a fusion protein with another protein, and the other protein interacts with a target substance. The "fusion protein," "other protein," and "target substance" as mentioned herein are the same as those described in the section "1. Method for Controlling vRNA Transcription and/or Replication by RdRp."

The other protein is not particularly limited, but is, for example, any one selected from the group consisting of a) a protein with specific binding affinity to a target substance, b) a light-responsive protein, c) an ion-responsive protein, d) a small molecule-binding protein, e) a nucleic acid-binding protein, and f) a temperature-responsive protein. Specific examples of these are described in the section "1. Method for Controlling vRNA Transcription and/or Replication by RdRp."

The positional relationship between the subunit and/or NP and the other protein in the fusion protein is as described in the section "1. Method for Controlling vRNA Transcription and/or Replication by RdRp"; however, it may be preferable to place the other protein on the N-terminal or C-terminal side of the subunit and/or NP, and it may be more preferable to configure a fusion protein with the other protein on the C-terminal side of the subunit and/or NP.

For example, the size of the other protein may be preferably 100 kDa or less, and more preferably 90 kDa or less, 80 kDa or less, 70 kDa or less, 65 kDa or less, 60 kDa or less, 55 kDa or less, 50 kDa or less, 45 kDa or less, or 40 kDa or less. The details are as described in the section "1. Method for Controlling vRNA Transcription and/or Replication by RdRp."

In a preferred embodiment, only one of the subunits constituting the modified RdRp is constructed as a fusion protein with another protein. The remaining two subunits may be wild-type or mutant. With regard to the subunit that is constructed as a fusion protein with another protein, the subunit portion may also be wild-type or mutant.

In a preferred embodiment, two or three of the subunits constituting the modified RdRp are constructed as fusion proteins with other different proteins. That is, two or three types of fusion proteins may be constructed. By constructing two or more types of fusion proteins, it is possible to target two or more types of target substances.

In a preferred embodiment, at least one of the subunits constituting the modified RdRp and NP constituting the modified NP are constructed as fusion proteins with other different proteins. Since the subunit and NP are constructed as fusion proteins with other proteins, it is possible to target two or more types of target substances. Furthermore, steric hindrance between the subunit and NP, and the other proteins that constitute the fusion proteins with each of them, is less likely to occur.

### 3. Nucleic Acid Construct and Composition Comprising Nucleic Acid Construct

The nucleic acid construct according to the present embodiment expresses an RNP-constituting protein that is constructed as a fusion protein with another protein and contained in the modified RNP-constituting protein described in the section "2. Modified RNP-Constituting Protein" above, or expresses a modified viral gene corresponding to the RNP-constituting protein.

The present embodiment also provides a composition for producing modified influenza virus, comprising the above nucleic acid construct. An example of this composition is a composition that produces modified influenza virus by the so-called reverse genetics method (Reference 1 listed in the Examples). For example, the composition is composed of the following (1) and (2).

(1) Nucleic acid constructs (e.g., plasmids) that express influenza virus genes (vRNAs). In the case of influenza A and B viruses, these correspond to eight types of plasmids corresponding to the eight segments that constitute the influenza virus gene. In the case of influenza C and D viruses, these correspond to seven types of plasmids corresponding to the seven segments that constitute the influenza virus gene.
(2) Nucleic acid constructs (e.g., plasmids) that express influenza virus proteins. Regardless of the type of influenza virus, these correspond to at least four types of plasmids that express proteins corresponding to the PA, PB1, PB2, and NP segments, generating mRNAs corresponding to each protein. In the case of influenza A and B viruses, nucleic acid constructs that express proteins corresponding to the eight segments mentioned above are more preferred. In the case of influenza C and D viruses, nucleic acid constructs that express proteins corresponding to the seven segments mentioned above are more preferred.

The nucleic acid constructs (1) and (2) may be the same nucleic acid construct. For example, the influenza virus genes and proteins corresponding to each of the eight or seven segments mentioned above may be expressed from a single nucleic acid construct.

When the modified RNP-constituting protein is modified RdRp, and the subunit constructed as a fusion protein with another protein is X (= PA, PB1, or PB2), the nucleic acid constructs (1) contain a plasmid that expresses, instead of the vRNA of X, modified vRNA corresponding to the fusion protein of X with another protein, and/or (preferably, and) the nucleic acid constructs (2) contain a plasmid that expresses, instead of X, the fusion protein of X with another protein. If other segments that maintain their function (e.g., wild-type) are expressed, infectious influenza virus particles can be formed within host cells.

Both the nucleic acid constructs (1) and (2) can be constructed to include known expression control sequences (e.g., promoters and terminators). For example, in the case of (1), usable promoters are an RNA polymerase I promoter, an RNA polymerase II promoter, an RNA polymerase III promoter, a T3 promoter, and a T7 promoter, and usable terminators are terminators corresponding to these promoters. In the case of (2), promoters suitable for the host cells may be selected. When the host cells are mammalian cells, for example, a CMV promoter and a CAG promoter can be used, and terminators corresponding to these promoters can be used.

The composition for producing modified influenza virus, comprising the above nucleic acid construct, can be used to control vRNA transcription and/or replication by influenza virus RdRp.

An embodiment of the present invention also includes a nucleic acid construct for controlling vRNA transcription and/or replication by RdRp, which expresses NP constructed as a fusion protein with another protein and contained in modified NP, in which NP of influenza virus is constructed as a fusion protein with another protein, and the other protein interacts with a target substance, or which expresses a modified viral gene corresponding to the NP.

### 4. Application

Although the uses of the nucleic acid constructs and compositions described in the section "3. Nucleic Acid Construct and Composition Comprising Nucleic Acid Construct" above are not particularly limited, they can be used, for example, for the production of influenza virus, as vectors, or for cell lysis. The influenza virus produced can be used, for example, for the production of vaccines. For use as vectors, they can express foreign genes inserted at appropriate sites of the plasmid within host cells, as with conventional influenza virus vectors. For use for cell lysis, viruses can lyse cells by replicating within the cells.

For example, if the subunit constituting RdRp and/or NP is a fusion protein with another protein, and the other protein is a protein that interacts with a target substance, the state of vRNA transcription and/or replication differs between cells with different amounts of the target substance. This can be used to induce or inhibit vRNA transcription and/or replication specifically in target cells. As a result, for use as vectors, foreign genes can be expressed specifically in target cells. For use for cell lysis, target cells can be specifically lysed by specifically replicating viruses within the target cells.

An example of the use as vectors is a vector for gene therapy targeting abnormal cells, and an example of the use for cell lysis is a cytolytic agent targeting abnormal cells. "Abnormal cells" refer to cells other than normal cells, such as cancer cells. In this case, the cytolytic agent corresponds to an oncolytic virus preparation. In particular, when abnormal cells frequently lack or have mutations in normal proteins or normal nucleic acid transcripts contained in normal cells, it is possible to specifically induce vRNA transcription and/or replication in the abnormal cells by using these normal proteins or normal nucleic acid transcripts as target substances, and employing other proteins that interact with these target substances. In addition, the scope of the present invention includes methods of treatment in which effective amounts of these pharmaceutical compositions are administered to subjects.

The method of administering the pharmaceutical composition is not particularly limited. The pharmaceutical composition may be locally administered by methods such as injection using a syringe or infusion pump, transdermal administration, or sublingual administration, or may be systemically administered by methods such as oral administration, intravascular administration into veins or arteries, or intestinal administration. In a preferred administration embodiment, the pharmaceutical composition is administered locally to the vicinity of the tissue to be treated.

The dosage (effective amount) of the pharmaceutical composition may be appropriately determined depending on the age, sex, symptoms, route of administration, frequency of administration, etc. of the human or animal as the target of administration. If necessary, the dosage can be determined by conducting an in vivo assay using the pharmaceutical composition in advance, without undue experimentation.

The frequency of administration of the pharmaceutical composition is also not particularly limited as long as the desired effect is obtained, and it may be appropriately set depending on, for example, the dosage, route of administration, symptoms, or the age and sex of the human or animal.

The pharmaceutical composition according to an embodiment of the present invention may contain at least the nucleic acid construct described above and a pharmaceutically acceptable carrier. The pharmaceutically acceptable carrier is not particularly limited, but preferably has the properties of not substantially inhibiting the function of the nucleic acid construct and not causing substantial adverse effects on the human or non-human animal as the target of administration. In some cases, it may be preferable for this pharmaceutical composition to contain a liquid carrier such as water, and one example may be a liposomal formulation.

Further examples of components constituting the above pharmaceutical composition include, but are not particularly limited to, lubricants, preservatives, stabilizers, humectants, emulsifiers, salts for adjusting osmotic pressure, buffers, colorants, antioxidants, viscosity adjusters, and the like.

### Summary

An embodiment of the present invention includes the following.
(1) A method for controlling vRNA transcription and/or replication by influenza virus RNA-dependent RNA polymerase (RdRp), comprising forming at least one selected from the group consisting of subunits constituting the RdRp and nucleoprotein (NP) into a fusion protein with another protein.
(2) The method according to (1), wherein at least one of the subunits constituting the RdRp is a fusion protein with another protein.
(3) The method according to (1) or (2), wherein the other protein interacts with a target substance.
(4) The method according to any one of (1) to (3), wherein the state of vRNA transcription and/or replication by RdRp and/or **NP** changes between a state in which the other protein and the target substance interact and a state in which they do not interact.
(5) Modified RdRp in which at least one of subunits constituting influenza virus RdRp is constructed as a fusion protein with another protein, and the other protein interacts with a target substance.
(6) The RNP-constituting protein according to (5), wherein the other protein is any one selected from the group consisting of 1) a protein with specific binding affinity to a target substance, 2) a light-responsive protein, 3) an ion-responsive protein, 4) a small molecule-binding protein, 5) a nucleic acid-binding protein, and 6) a temperature-responsive protein.
(7) The RNP-constituting protein according to (5) or (6), wherein the subunit is constructed as a fusion protein with the other protein at the C-terminus of the subunit.
(8) The RNP-constituting protein according to any one of (5) to (7), wherein the other protein is 100 kDa or less in size.
(9) A nucleic acid construct that expresses the subunit and/or **NP** constructed as a fusion protein with another protein and contained in the modified RNP-constituting protein of any one of (5) to (7), or that expresses a modified viral gene corresponding to the subunit.
(10) The nucleic acid construct according to (9), which is a nucleic acid construct for controlling vRNA transcription and/or replication by RdRp.
(11) A nucleic acid construct for controlling vRNA transcription and/or replication by RdRp, which expresses NP constructed as a fusion protein with another protein and contained in modified NP, in which NP of influenza virus is constructed as a fusion protein with another protein, and the other protein interacts with a target substance, or which expresses a modified viral gene corresponding to the NP.
(12) A composition for producing modified influenza virus, comprising the nucleic acid construct of any one of (9) to (11).
(13) The composition according to (12), for use in vectors or cell lysis.

The present invention is not limited to the embodiments described above, and various modifications are possible within the scope of the claims. Embodiments obtained by appropriately combining the technical means disclosed in the different embodiments are also included in the technical scope of the present invention.

### Examples

Examples of the present invention are described below.

### Example 1

### Materials and Methods

### Cells and Viruses

293T human embryonic kidney (HEK293T), Madin-Darby canine kidney (MDCK), Sarcoma osteogenic (Saos-2), and HeLa S3 cells were cultured in Dulbecco's modified Eagle's medium (DMEM, Fujifilm) supplemented with 10% fetal calf serum (FCS, Japan Bio Serum), 100 units/mL penicillin, and 100 mg/mL streptomycin (Fujifilm) at 37°C in 5% CO₂. All viruses used in this Example were based on influenza A/Puerto Rico/8/1934 (PR8) and generated using reverse genetics (Reference 1).

### Construction of Plasmids

All plasmids used in this Example were constructed by standard PCR and seamless cloning methods. The PA, PB1, PB2, or NP segment of PR8 was subcloned into the pcDNA3 plasmid, and pcDNA3-PA, pcDNA3-PB1, pcDNA3-PB2, or pcDNA3-NP was designed. Plasmid of PA, PB1, or PB2 fused to the protein (X), strep-tag, or Nb139 at the N-terminus or C-terminus was obtained by cloning the sequence of the corresponding protein into pcDNA3-PA, pcDNA3-PB1, or pcDNA3-PB2. pPolI plasmid was constructed by inserting the synthetic human RNA polymerase I promoter (p_{I}) and the mouse RNA polymerase I terminator (t_{I}) sequences into pcDNA3 from which the CMV promoter (CMV) and BGH polyadenylation signal (pA) sequences had been removed. pPolI-mScarlet was constructed by inserting the mScarlet-I sequence between p_{I} and t_{I} of the pPolI plasmid in the negative-sense orientation. To obtain pcDNA3-streptavidin-mTurquoise plasmid, two PCR-fragments including the sequence of streptavidin and the sequence of mTurquoise2 with the porcine teschovirus 2A (P2A) self-cleavage site (ATNFSLLKQAGDVEENPGP: SEQ ID NO: 1) at the N-terminus were inserted into pcDNA3. To construct pcDNA3-PolI plasmid, synthetic p_{I} and t_{I} sequences were inserted between CMV and pA of pcDNA3 in the negative-sense orientation. The eight plasmids for generating influenza virus were created by inserting the sequence of the corresponding segment between t_{I} and p_{I} of pcDNA-PolI in the positive-sense orientation, and pcDNA3-PolI-PA, pcDNA3-PolI-PB1, pcDNA3-PolI-PB2, pcDNA3-PolI-HA, pcDNA3-PolI-NP, pcDNA3-PolI-NA, pcDNA3-PolI-M, and pcDNA3-PolI-NS were designed. pcDNA3-PolI-PB1-Nb139 was constructed by inserting the Nb139 sequence followed by packaging signal (PS) that is 88 nucleotides of the original noncording and cording sequences in the PB1 segment. mScarlet-I gene was cloned between the NS1 and nuclear export protein (NEP) sequences of pcDNA3-PolI-NS to construct pcDNA3-PolI-NS-mScarlet plasmid (Reference 2). The splice acceptor site in NS was mutated to avoid original splicing. The sequence of P2A site was followed by the NS1 sequence to generate NS1-mScarlet-I protein and NEP protein individually. All plasmids were confirmed by DNA sequencing.

### RNP Activity Assay

The HEK293T cells were seeded one day prior to transfection on a 3.5 cm glass bottom or normal culture dish with 5 × 10⁵ cells/2 mL. 0.7 µg of each of pcDNA-PA (-X-PA or PA-X), pcDNA-PB1 (-X-PB1 or PB1-X), and pcDNA-PB2 (-X-PB2 or PB2-X) plasmids was co-transfected with 0.6 µg of pcDNA-NP plasmid and 0.3 µg of pPolI-mScarlet plasmid using Lipofectamine LTX (Thermo Fisher Science) and Opti-MEM (Thermo Fisher Science) according to the manufacturer's instructions. At 48 hours post-transfection (hpt), red fluorescence images were taken using a confocal laser scanning microscope (LSM 710, Carl Zeiss), and the cells were lysed in 100 µl of lysis buffer (50 mM Tris-HCl, pH: 8.0, 200 mM NaCl, 0.5% Nonidet P-40 (Sigma-Aldrich), 1 mM DTT, 1 mM PMSF, 25% glycerol, and 1 x protease inhibitor cocktail (Fujifilm)) at 4°C for 60 minutes. The lysates were analyzed using a fluorescence microplate reader (SH-9000, Hitachi).

### RNP Formation Assay

The HEK293T cells were seeded one day prior to transfection on a 10 cm culture dish with 4 × 10⁶ cells/10 mL. 2.7 µg of each of pcDNA-PA (-X-PA or PA-X), pcDNA-PB1 (-X-PB1 or PB1-X), and pcDNA-PB2 (-X-PB2 or PB2-X) plasmids was co-transfected with 2.5 µg of pcDNA-NP plasmid and 1.3 µg of pPolI-mScarlet plasmid into the HEK293T cells using Lipofectamine LTX and Opti-MEM. The sequence encoding twin-strep-tag (WSHPQFEKGGGSGGGSGGSAWSHPQFEK: SEQ ID NO: 2) was inserted into X-PA (or PA-X), X-PB1 (or PB1-X), and X-PB2 (or PB2-X) before (or after) the sequence encoding the protein X. At 48 hpt, the cells were lysed in 500 µl of lysis buffer at 4°C for 60 minutes. To the lysate in 2.5 mL of binding buffer (100 mM Tris-HCl, pH: 8.0 and 150 mM NaCl), 50 mL of Strep-Tactin XT 4Flow (IBA) was added and incubated at 4°C for 3 hours. The Strep-Tactin XT 4Flow was then washed three times with 3 mL of wash buffer (100 mM Tris-HCl, pH: 8.0, 150 mM NaCl, 0.1% Nonidet P-40, 1 mM PMSF, and 10% glycerol). RNP complexes were released from the Strep-Tactin XT 4Flow by elution buffer (100 mM Tris-HCl, pH: 8.0, 150 mM NaCl, 50 mM biotin, 0.1% Nonidet P-40, 0.1% glycerol, 1 mM DTT, 1 mM PMSF, and 1 x protease inhibitor cocktail) at 4°C for 1 hour, and analyzed by 7.5% SDS-PAGE followed by silver staining.

### RNP Activity Assay for Exogenous Streptavidin

The HEK293T cells were seeded one day prior to transfection on a 3.5 cm glass bottom or normal culture dish with 5 × 10⁵ cells/2 mL. 0.23 µg of each of pcDNA-PA (-strep-PA or PA-strep), pcDNA-PB1 (-strep-PB1 or PB1-strep), and pcDNA-PB2 (-strep-PB2 or PB2-strep) plasmids was co-transfected with 0.2 µg of pcDNA-NP plasmid and 0.1 µg of pPolI-mScarlet plasmid in the presence or absence of 2 µg of pcDNA3-streptavidin-mTurquoise plasmid using Lipofectamine LTX and Opti-MEM. At 48 hpt, red and cyan fluorescence images were taken using LSM, and the cells were lysed in 100 µl of lysis buffer at 4°C for 60 minutes. The lysates were analyzed using a fluorescence microplate reader.

### RNP Activity Assay for Endogenous p53

The HEK293T and Saos-2 cells were seeded one day prior to transfection on a 3.5 cm glass bottom or normal culture dish with 5 × 10⁵ cells/2 mL and 1 × 10⁶ cells/2 mL, respectively. 110 ng of each of pcDNA-PA (-Nb139-PA or PA-Nb139), pcDNA-PB1 (-Nb139-PB1 or PB1-Nb139), and pcDNA-PB2 (-Nb139-PB2 or PB2-Nb139) plasmids was co-transfected with 100 ng of pcDNA-NP and 70 ng of pPolI-mScarlet plasmid using Lipofectamine LTX and Opti-MEM. For addition of excess Nb139 protein as a decoy, 800 ng of pcDNA3-Nb139 plasmid was co-transfected with the earlier plasmids. At 48 hpt, red fluorescence images were taken using LSM, and the cells were lysed in 100 µl of lysis buffer at 4°C for 60 minutes. The lysates were analyzed using a fluorescence microplate reader.

### Generation of Influenza Virus and Infection

The HEK293T and MDCK cells were seeded one day prior to transfection on one 3.5 cm culture dish with 2.5 x 10⁵ cells/2 ml each. 0.3 µg of each of the eight pcDNA-PolI plasmids was co-transfected with 0.7 µg of pcDNA-Nb139 using Lipofectamine LTX and Opti-MEM. For fluorescence reporter assay, pcDNA-PolI-NS-mScarlet plasmid was transfected instead of pcDNA-PolI-NS plasmid. At 24 hpt, the cells were washed twice with phosphate-buffered saline (PBS), the medium was then replaced with DMEM containing 0.2% bovine serum albumin (BSA, Sigma-Aldrich), 100 units/mL penicillin, 100 mg/mL streptomycin, and 1 mg/mL N-p-Tosyl-L-phenylalanine chloromethyl ketone (TPCK)-treated trypsin (Sigma-Aldrich), and the cells were cultured at 37°C in 0.5% CO₂ for 2 days to produce IAV. The MDCK, HEK293T, Saos-2, and HeLa S3 cells were infected with IAV at MOIs of 0.1, 1, 1, and 1, respectively, in an infection medium (DMEM containing 0.2% BSA, 100 units/mL penicillin, 100 mg/mL streptomycin, and 0.5 mg/mL TPCK-treated trypsin). At 24 hours post-infection (hpi), the cells were fixed and permeabilized with 3.7% formaldehyde (Fujifilm) and 0.5% Triton X-100 (Sigma-Aldrich) in PBS at room temperature for 15 minutes. The cells were washed twice with PBS before being treated with blocking buffer (2.5% BSA in PBS) at room temperature for 1 hour. After washing with PBS, the cells were incubated with 1 µg/mL of anti-NP mouse monoclonal antibody (Sino Biological) at 37°C for 1 hour. Then, the cells were washed with PBS, and incubated with 6 µg/mL of FITC-conjugated goat anti-mouse IgG secondary antibody (Proteintech) at 37°C for 1 hour. Green and red fluorescence images were taken using LSM.

### Growth Kinetics

The MDCK, HEK293T, Saos-2, and HeLa S3 cells were infected with IAV at MOIs of 0.01, 0.1, 5, and 5, respectively, in an infection medium at 37°C for 1 hour. The cells were then washed with PBS, and fresh infection medium was added. The supernatant of each infected cell was sampled at the indicated time points. 1% guinea-pig red blood (Cedarlane) was mixed with the virus sample two-fold serially diluted in PBS, and incubated at 4°C for 45 minutes. The highest agglutinating well was read as the HA titer.

### Plaque Assay

The MDCK cells were seeded one day prior to titration on a 3.5 cm culture dish with 5 × 10⁵ cells/2 mL. The cells were washed twice with PBS and then infected with virus ten-fold serially diluted in DMEM at 37°C for 1 hour with agitation every 10 minutes. The inoculated virus was removed before the addition of an infection medium containing 0.5% ager. The cells were incubated at 37°C in 0.5% CO₂ humidified atmosphere for 72 hours. Then, the cells were fixed with 3.7% formaldehyde in PBS at room temperature for 60 minutes. The cells were washed twice with PBS, and the plaques were visualized with 1% crystal violet.

### Pull-Down Assay

The HEK293T and Saos-2 cells were seeded one day prior to transfection on a 10 cm culture dish with 4 × 10⁶ cells/10 mL and 8 × 10⁶ cells/10 mL, respectively. 1.1 µg of each of pcDNA-PA, pcDNA-PB1 (or PB1-Nb139), and pcDNA-PB2 plasmids was co-transfected with 0.6 µg of pPolI-mScarlet plasmid in the presence or absence of 0.9 µg of pcDNA-NP plasmid. Twin-strep-tag was inserted into the C-terminus of the PA subunit. For addition of excess Nb139 protein as a decoy, 7.4 µg of pcDNA3-Nb139 plasmid was co-transfected with the earlier plasmids. At 48 hours post-transfection (48 hpt), the cells were lysed in 500 µl of lysis buffer at 4°C for 60 minutes. Strep-Tactin XT 4Flow (50 µl) was added to the lysate in 2.5 mL of binding buffer, followed by incubation at 4°C for 3 hours. The Strep-Tactin XT 4Flow was then washed three times with 3 mL of wash buffer. RNP complexes were released from the Strep-Tactin XT 4Flow by elution buffer at 4°C for 1 hour, and analyzed by 10% SDS-PAGE followed by silver staining.

### Results etc.

### Influence of Modified RdRp on RNP Activity

To systematically examine the relationship between RNP activity and the size of the protein fused to PA, PB1, or PB2, 24 types of plasmids expressing the following proteins fused to one of PA, PB1, or PB2 were prepared: IgG-binding domain B domain of protein A (Protein A; 6.8 kDa), NanoLuc (Nluc; 19.1 kDa), Renilla luciferase (Rluc; 36.0 kDa), or cryptochrome 2 (CRY2; 57.2 kDa) (Fig. 2(a)). The proteins were fused at the N-terminus or C-terminus through a glycine-serine (GS) linker and were incorporated in RNPs in culture cells. Plasmids for the construction of RdRp, wherein one subunit was fused to one of the four proteins mentioned above, were co-transfected into HEK293T cells with a plasmid for NP and a pPolI-mScarlet plasmid that expresses negative-sense RNA of the red fluorescent protein, mScarlet-I (Fig. 2(b)). The resultant RNP included negative-sense RNA of mScarlet-I that produced mScarlet-I protein via transcription and replication, such that RNA activity could be monitored by the red fluorescence intensity. Fluorescence intensities from cell lysates are summarized in Fig. 2(c). Strong red fluorescence intensity was observed in cells expressing wild-type RNP, whereas no fluorescence was observed in the absence of NP, indicating that NP is essential for transcription and replication. In lysates from cells transfected with the fusion protein, the fluorescence intensity decreased as the size of the fusion protein increased. In particular, the fluorescence intensity was significantly reduced when proteins other than Protein A were fused at the N-terminus of PA or PB1, suggesting that proteins with a molecular weight of more than 6.8 kDa significantly interfere with transcription and replication processes. Since the molecular weight of nanobodies is 12 to 15 kDa, these two candidates may be insufficient for the method of the inventors. However, the incorporation of a protein at the C-terminus of PA and PB2 was relatively tolerated, yielding fluorescence intensities of approximately 40 to 50% of the wild-type level, even when CRY2, the largest protein, was fused. Protein fusion at the C-terminus of PB1 revealed a moderate reduction in fluorescence (Fig. 2B). Next, the RNP formation assay was performed to examine why fusion of larger-sized proteins on the RdRp subunits inhibited RNP activity. RNP was extracted and purified from HEK293T cells and analyzed by gel electrophoresis. Clear bands corresponding to the three subunits of RdRp were observed with or without the band for NP, indicating that the modified subunit can form a stable heterotrimeric RdRp with other wild-type subunits (Fig. 2A). With the increase in the size of the fusion protein, the accumulation of NP into RNP decreased, coinciding with the decrease of red fluorescence from mScarlet-I. These results demonstrate that fusion of the PA, PB1, or PB2 subunit with a large protein inhibits RNP formation by preventing NP uptake without inhibiting assembly into the heterotrimeric RdRp. The prevention of NP-accumulation into RNP is likely caused by the steric hindrance between NP and the fusion protein. Because NP interacts with all three RdRp subunits in a different manner and maintains the assembly of RNP, the extent of steric hindrance depends on the site of protein fusion. In particular, because NP binds to residues 1 to 27 of PA, significant steric hindrance was observed between the protein fused to the N-terminus of PA and NP, even though the fusion protein was small.

### Regulation of RNP Activity by Interaction System

To verify whether RNP activity can be regulated by the protein interaction system, the subunits of RdRp were fused to a strep-tag that can strongly and specifically bind to streptavidin (Reference 3) (Fig. 3(b)). Strep-tag is a relatively small peptide, and its fusion to the RdRp subunits does not interfere with RNP formation. However, in the presence of a streptavidin tetramer (53 kDa), the strep-tag site on the subunit becomes bulky, inhibiting the formation of RNP (Fig. 3(a)). To assess the behavior of this system in HEK293T cells, plasmids for RNP construction were co-transfected with the pPolI-mScarlet plasmid and a plasmid consisting of an ORF of streptavidin followed by an ORF of mTurquoise2 separated by a P2A site, which allowed the monitoring of streptavidin expression by cyan fluorescence from the co-expressed mTurquoise2. In wild-type RNP, red fluorescence levels did not significantly differ in the presence or absence of streptavidin, suggesting that streptavidin overexpression does not affect RNP activity (Fig. 3(c) and Fig. 3A). Conversely, a significant difference in red fluorescence was observed based on the presence or absence of streptavidin when the RdRp subunits were fused to a Strep-tag, particularly in the case of PB1 fused to a Strep-tag at its C-terminus, which resulted in a 24.9-fold reduction in fluorescence intensity.

### Regulation of RNP Activity using Nanobody

To confirm the detectability of endogenous cellular proteins, tumor suppressor protein p53, which is frequently deleted or mutated in malignant tumor cells, and a nanobody (Nb139) that can specifically bind to the DNA-binding domain (DBD) of p53 were selected (Reference 4). Nanobodies derived from camelids are the smallest naturally occurring antibodies. Nb139 has a molecular weight of 13.4 kDa. Thus, fusing Nb139 to the subunits of RdRp, except for fusion at the N-terminus of PA or PB1, should not considerably influence RNP activity. However, when endogenous p53 (53 kDa) binds to Nb139 that is fixed to the subunit of RdRp, NP accumulation becomes insufficient, resulting in reduced RNP activity (Fig. 4(a)). The inventors of the present application prepared plasmids for PA, PB1, or PB2 with Nb139 fused to the N-terminus or C-terminus through a GS linker, and tested them separately in HEK293T cells expressing wild-type p53 and in Saos-2 cells lacking p53 (Fig. 4(b)). Plasmids for each Nb139-fused subunit, the remaining subunits (wild-type) of RdRp, and pPolI-mScarlet were co-transfected into each cell line. In the Saos-2 cells, the activity of RNP was decreased owing to the fusion of Nb139; conversely, the activity of RNP was decreased dramatically in the HEK293T cells, likely because of the interaction of endogenous p53 with Nb139 in the RNP complex. In this experiment, the fusion of Nb139 to the C-terminus of PB1 showed the most dramatic effect, amounting to a 12.9-fold difference in red fluorescence between the HEK293T and Saos-2 cells (Figs. 4 (c) and (d)). To confirm that the interaction between endogenous p53 and Nb139 fused to the C-terminus of PB1 was the reason for the drastic decrease in RNP activity, Nb139 was overexpressed in these cells. In consequence, the expression levels of free Nb139 are considered to act as a decoy and outcompete the subunit fused to Nb139 for the DBD of p53. In this situation, the RNP activity was expected to recover in the HEK293T cells owing to Nb139 overexpression. No changes in RNP activity were observed in the Saos-2 or HEK293T cells containing wild-type RNP, even when Nb139 was overexpressed. In contrast, the activity of RNP containing Nb139-fused PB1 in the HEK293T cells significantly recovered with Nb139 overexpression (Fig. 4e). Additionally, a pull-down assay was performed on p53 showing that the protein was not detected in Saos-2, but was observed in the HEK293T cells. The decline in the intensity of the band corresponding to NP indicated that the accumulation of NP into RNP was inhibited by the interaction of p53 and Nb139 fused to the C-terminus of PB1 (Fig. 4(f)). Overexpression of Nb139 caused the disappearance of the p53 band and reappearance of the NP band. These results suggest that the decrease in the activity of PB1-Nb139-containing RNP in the HEK293T cells is caused by the interaction of endogenous p53 and Nb139 fused to the C-terminus of PB1.

### Generation of Replication-Competent IAV

The inventors of the present application generated replication-competent IAV containing PB1-Nb139 using an eight-plasmid system (Fig. 5(a)). The eight plasmids were co-transfected into a co-culture system of HEK293T and MDCK cells with an Nb139-expression plasmid to mask endogenous p53 and to efficiently generate PB1-Nb139-containing IAV. Endogenous p53 was masked because both HEK293T and MDCK cells express wild-type p53. The virus titers of recombinant IAVs were measured by plaque formation in MDCK cells, and the MOI was calculated from the virus titer. Because MDCK cells express wild-type p53, causing the inhibition of RNP activity, determination of the virus titer of IAV containing PB1-Nb139 was not possible. As a substitute, the virus titer was measured for IAV that contained PB1 fused to LaM4, a nanobody for a red fluorescent protein (Reference 5). There was no difference in the results of the HA assay between the IAV containing PB1-LaM4 and IAV containing PB1-Nb139. Hence, this substitution to assess the virus titer was appropriate. p53-expressing cell lines HEK293T and MDCK, or p53-deficient cell lines Saos-2 and HeLa S3, were infected with wild-type IAV or IAV containing PB1-Nb139. Viral infection and replication were confirmed by red fluorescence from mScarlet-I fused to NS1 protein and by the expression of NP visualized by anti-NP MAb (Fig. 5b). In the Saos-2 and HeLa S3 cells, no significant differences were observed in fluorescence between cells infected with wild-type IAV and those infected with IAV containing PB1-Nb139. However, strong fluorescence was observed in the HEK293T and MDCK cells infected with wild-type IAV, whereas no fluorescence was observed when PB1-NB139-containing IAV was used, indicating that PB1-NB139-containing IAV did not replicate in those cells. Next, the growth characteristics of PB1-Nb139-containing IAV were compared with those of wild-type IAV in all four cell lines using the HA assay (Fig. 5(c)). The difference in peak HA titers between wild-type IAV and PB1-Nb139-containing IAV was approximately 2-fold in the Saos-2 and HeLa S3 cells, and approximately 2⁵-fold in the HEK293T and MDCK cells. The fluorescence images and growth characteristics revealed that IAV containing PB1-Nb139 can replicate only in cells not containing p53. mScarlet-I was inserted as a reporter into the NS segment, demonstrating that the foreign gene mScarlet-I was expressed in a cell type-specific manner by a replication-competent IAV.

### Example 2

### Generation of Influenza Virus Containing LaM4 and Infection

Plasmid pcDNA3-PolI-PB1-LaM4, in which LaM4 was fused to the C-terminus of PB1, was constructed by standard PCR and seamless cloning methods. The sequence was obtained by replacing Nb139 of the plasmid for PB1 in Fig. 5(a) with LaM4. The HEK293T and MDCK cells were seeded one day prior to transfection on one 3.5 cm culture dish with 2.5 x 10⁵ cells/2 ml each. 0.35 µg of each of the eight pcDNA-PolI plasmids was co-transfected using Lipofectamine LTX and Opti-MEM. At 24 hpt, the cells were washed twice with phosphate-buffered saline (PBS), the medium was then replaced with DMEM containing 0.2% bovine serum albumin (BSA, Sigma-Aldrich), 100 units/mL penicillin, 100 mg/mL streptomycin, and 1 mg/mL N-p-Tosyl-L-phenylalanine chloromethyl ketone (TPCK)-treated trypsin (Sigma-Aldrich), and the cells were cultured at 37°C in 0.5% CO₂ for 2 days to produce PB1-LaM4-containing IAV. The MDCK cells and DsRed-expressing MDCK cells were infected with wild-type IAV or PB1-LaM4-containing IAV at an MOI of 0.1 in an infection medium (DMEM containing 0.2% BSA, 100 units/mL penicillin, 100 mg/mL streptomycin, and 0.5 mg/mL TPCK-treated trypsin). At 48 hours post-infection, the cells were washed with PBS and stained with 1% crystal violet. In addition, the MDCK cells and DsRed-expressing MDCK cells were infected with wild-type IAV or PB1-LaM4-containing IAV at an MOI of 0.01, and the growth characteristics over 3 days were measured using the HA assay.

### Generation of Replication-Competent IAV Containing LaM4

In the MDCK cells not expressing DsRed, cell degeneration (apoptosis) due to the replication of PB1-LaM4-containing IAV was observed 2 days after infection (Fig. 6(b)). On the other hand, no cell degeneration was observed in the MDCK cells expressing DsRed. These results indicate that the binding of DsRed to LaM4 inhibited the replication of replication-competent IAV containing PB1-LaM4. Next, the growth characteristics of IAV containing PB1-LaM4 were compared with those of wild-type IAV using the HA assay (Fig. 6(c)). In the MDCK cells expressing DsRed, the difference in peak HA titers between wild-type IAV and IAV containing PB1-LaM4 was 2⁵-fold. On the other hand, in the MDCK cells not expressing DsRed, the difference was 2²-fold. These results revealed that IAV containing PB1-LaM4 can replicate only in cells not containing DsRed.

### Example 3

### Generation of Influenza Virus Containing Vivid and Infection

Plasmid pcDNA3-PolI-PB1-vivid, in which vivid was fused to the C-terminus of PB1, was constructed by standard PCR and seamless cloning methods. The sequence was obtained by replacing Nb139 of the plasmid for PB1 in Fig. 5(a) with vivid. The HEK293T and MDCK cells were seeded one day prior to transfection on one 3.5 cm culture dish with 2.5 x 10⁵ cells/2 ml each. 0.35 µg of each of the eight pcDNA-PolI plasmids was co-transfected using Lipofectamine LTX and Opti-MEM. At 24 hpt, the cells were washed twice with phosphate-buffered saline (PBS), the medium was then replaced with DMEM containing 0.2% bovine serum albumin (BSA, Sigma-Aldrich), 100 units/mL penicillin, 100 mg/mL streptomycin, and 1 mg/mL N-p-Tosyl-L-phenylalanine chloromethyl ketone (TPCK)-treated trypsin (Sigma-Aldrich), and the cells were cultured at 37°C in 0.5% CO₂ for 2 days to produce PB1-vivid-containing IAV. The cells were infected with PB1-vivid containing IAV in an infection medium (DMEM containing 0.2% BSA, 100 units/mL penicillin, 100 mg/mL streptomycin, and 0.5 mg/mL TPCK-treated trypsin). One of the two culture dishes infected with PB1-vivid-containing IAV was intermittently irradiated with 460 nm light (10 seconds of light irradiation, 1-minute interval). At 48 hours post-infection, the cells were washed with PBS and stained with 1% crystal violet. In addition, the MDCK cells were infected with wild-type IAV or PB1-vivid-containing IAV, and the growth characteristics over 3 days, with and without light irradiation, were measured using the HA assay.

### Generation of Replication-Competent IAV Containing Vivid

In the MDCK cells that were not irradiated with light, cell degeneration (apoptosis) due to the replication of PB1-vivid-containing IAV was observed (Fig. 7(b)). On the other hand, no cell degeneration was observed in the MDCK cells irradiated with light. These results indicate that light irradiation induces vivid homodimerization, thereby inhibiting the replication of replication-competent IAV containing PB1-vivid. Next, the growth characteristics of IAV containing PB1-vivid were compared with those of wild-type IAV using the HA assay (Fig. 7(c)). In the absence of light irradiation, the difference in peak HA titers between wild-type IAV and IAV containing PB1-vivid was 2⁴-fold. On the other hand, in the presence of light irradiation, the difference was 2⁵-fold. These results revealed that IAV containing PB1-vivid can replicate only when it is not irradiated with 460 nm blue light.

### Example 4

### Generation of Influenza Virus Containing Affibody and Infection

Plasmid pcDNA3-PolI-FcAffi-PB1 or pcDNA3-PolI-PB1-FcAffi, in which FcAffi, which is an Affibody specifically binding to the Fc region of IgG, was fused to the N-terminus or C-terminus of PB1, was constructed by standard PCR and seamless cloning methods. The HEK293T and MDCK cells were seeded one day prior to transfection on one 3.5 cm culture dish with 2.5 x 10⁵ cells/2 ml each. 0.35 µg of each of the eight pcDNA-PolI plasmids was co-transfected using Lipofectamine LTX and Opti-MEM. At 24 hpt, the cells were washed twice with phosphate-buffered saline (PBS), the medium was then replaced with DMEM containing 0.2% bovine serum albumin (BSA, Sigma-Aldrich), 100 units/mL penicillin, 100 mg/mL streptomycin, and 1 mg/mL N-p-Tosyl-L-phenylalanine chloromethyl ketone (TPCK)-treated trypsin (Sigma-Aldrich), and the cells were cultured at 37°C in 0.5% CO₂ for 2 days to produce FcAffi-PB1- or PB1-FcAffi-containing IAV. MDCK cells not expressing IgG Fc region proteins and MDCK cells expressing IgG Fc region proteins were infected with wild-type IAV or FcAffi-PB1- or PB1-FcAffi-containing IAV at an MOI of 0.1 in an infection medium (DMEM containing 0.2% BSA, 100 units/mL penicillin, 100 mg/mL streptomycin, and 0.5 mg/mL TPCK-treated trypsin). At 48 hours post-infection, the cells were washed with PBS and stained with 1% crystal violet. In addition, MDCK cells not expressing IgG Fc region proteins and MDCK cells expressing IgG Fc region proteins were infected with wild-type IAV or FcAffi-PB1- or PB1-FcAffi-containing IAV at an MOI of 0.01, and the growth characteristics over 3 days were measured using the HA assay.

### Generation of Replication-Competent IAV Containing Affibody

In the MDCK cells not expressing the Fc region of IgG, cell degeneration (apoptosis) due to the replication of FcAffi-PB1- or PB1-FcAffi-containing IAV was observed 2 days after infection (Fig. 8(c)). On the other hand, in the MDCK cells expressing IgG Fc region proteins, cell degeneration was observed when infected with FcAffi-PB1-containing IAV; however, the degree of cell degeneration was lower than when the MDCK cells not expressing IgG Fc region proteins were infected. When the MDCK cells expressing the Fc region of IgG were infected with PB1-FcAffi-containing IAV, only slight cell degeneration was observed. These results indicate that the binding of the IgG Fc region protein to FcAffi inhibited the replication of replication-competent IAV containing FcAffi-PB1 or PB1-FcAffi. Next, the growth characteristics of IAV containing FcAffi-PB1 or PB1-FcAffi were compared with those of wild-type IAV using the HA assay (Fig. 8(d)). In the MDCK cells expressing IgG Fc region proteins, the difference in peak HA titers between wild-type IAV and IAV containing FcAffi-PB1 was 2²-fold. On the other hand, in the MDCK cells not expressing IgG Fc region proteins, there was no difference. Further, in the MDCK cells expressing IgG Fc region proteins, the difference in peak HA titers between wild-type IAV and IAV containing PB1-FcAffi was 2⁵-fold. On the other hand, in the MDCK cells not expressing IgG Fc region proteins, there was no difference. These results revealed that IAV containing FcAffi-PB1 or PB1-FcAffi can replicate efficiently in cells that do not contain IgG Fc region proteins.

### Example 5

### Generation of Influenza Virus with Enhanced Replication Ability upon Binding to Target Substance

Plasmid pcDNA3-PolI-PB2-DegNb7, in which DegNb7, which is a nanobody specifically binding to EGFP, was fused to the C-terminus of PB2, was constructed by standard PCR and seamless cloning methods. The HEK293T cells and MDCK cells expressing EGFP were seeded one day prior to transfection on one 3.5 cm culture dish with 2.5 x 10⁵ cells/2 ml each. 0.35 µg of each of the eight pcDNA-PolI plasmids and 1 µg of pCDNA3-EGFP plasmid for EGFP expression were transfected using Lipofectamine LTX and Opti-MEM. At 24 hpt, the cells were washed twice with phosphate-buffered saline (PBS), the medium was then replaced with DMEM containing 0.2% bovine serum albumin (BSA, Sigma-Aldrich), 100 units/mL penicillin, 100 mg/mL streptomycin, and 1 mg/mL N-p-Tosyl-L-phenylalanine chloromethyl ketone (TPCK)-treated trypsin (Sigma-Aldrich), and the cells were cultured at 37°C in 0.5% CO₂ for 2 days to produce PB2-DegNb7-containing IAV. MDCK cells not expressing EGFP and MDCK cells expressing EGFP were infected with wild-type IAV or PB2-DegNb7-containing IAV at an MOI of 0.1 in an infection medium (DMEM containing 0.2% BSA, 100 units/mL penicillin, 100 mg/mL streptomycin, and 0.5 mg/mL TPCK-treated trypsin). At 48 hours post-infection, the cells were washed with PBS and stained with 1% crystal violet. In addition, MDCK cells not expressing EGFP and MDCK cells expressing EGFP were infected with wild-type IAV or PB2-DegNb7-containing IAV at an MOI of 0.01, and the growth characteristics over 3 days were measured using the HA assay.

### Generation of Replication-Competent IAV with Enhanced Replication Ability upon Binding to Target Substance

In the MDCK cells expressing EGFP, cell degeneration (apoptosis) due to the replication of PB2-DegNb7-containing IAV was observed 2 days after infection (Fig. 9(b)). On the other hand, slight cell degeneration was observed in the MDCK cells not expressing EGFP. These results indicate that the binding of EGFP to DegNb7 enhanced the replication ability of replication-competent IAV containing PB2-DegNb7. Next, the growth characteristics of PB2-DegNb7-containing IAV were compared with those of wild-type IAV using the HA assay (Fig. 9(c)). In the MDCK cells expressing EGFP, the difference in peak HA titers between wild-type IAV and PB2-DegNb7-containing IAV was 2²-fold. On the other hand, in the MDCK cells not expressing EGFP, the difference was 2⁵-fold. These results revealed that PB2-DegNb7-containing IAV can replicate efficiently in cells containing

### EGFP.

### Example 6

### Generation of Influenza Virus with NP Fused to Nanobody and Infection

Plasmid pcDNA3-PolI-DegNb8-NP or pcDNA3-PolI-NP-DegNb8, in which DegNb8, which is a nanobody specifically binding to EGFP, was fused to the N-terminus or C-terminus of NP, was constructed by standard PCR and seamless cloning methods. The HEK293T and MDCK cells were seeded one day prior to transfection on one 3.5 cm culture dish with 2.5 x 10⁵ cells/2 ml each. When preparing DegNb8-NP-containing IAV, MDCK cells expressing EGFP were seeded. 0.35 µg of each of the eight pcDNA-PolI plasmids was co-transfected using Lipofectamine LTX and Opti-MEM. When preparing DegNb8-NP-containing IAV, 1 µg of pcDNA3-EGFP plasmid for EGFP expression was co-transfected. At 24 hpt, the cells were washed twice with phosphate-buffered saline (PBS), the medium was then replaced with DMEM containing 0.2% bovine serum albumin (BSA, Sigma-Aldrich), 100 units/mL penicillin, 100 mg/mL streptomycin, and 1 mg/mL N-p-Tosyl-L-phenylalanine chloromethyl ketone (TPCK)-treated trypsin (Sigma-Aldrich), and the cells were cultured at 37°C in 0.5% CO₂ for 2 days to produce DegNb8-NP- or NP-DegNb8-containing IAV. MDCK cells not expressing EGFP and MDCK cells expressing EGFP were infected with wild-type IAV or IAV containing DegNb8-NP or NP-DegNb8 at an MOI of 1 in an infection medium (DMEM containing 0.2% BSA, 100 units/mL penicillin, 100 mg/mL streptomycin, and 0.5 mg/mL TPCK-treated trypsin). At 48 hours post-infection, the cells were washed with PBS and stained with 1% crystal violet. In addition, MDCK cells not expressing EGFP and MDCK cells expressing EGFP were infected with wild-type IAV or DegNb8-NP- or NP-DegNb8-containing IAV at an MOI of 0.1, and the growth characteristics over 3 days were measured using the HA assay.

### Generation of Replication-Competent IAV with NP Fused to Nanobody

When infected with DegNb8-NP-containing IAV, in the MDCK cells not expressing EGFP, cell degeneration (apoptosis) was observed 2 days after infection, whereas only slight cell degeneration was observed in the MDCK cells not expressing EGFP (Fig. 10(c)). On the other hand, when infected with NP-DegNb8-containing IAV, in the MDCK cells not expressing EGFP, cell degeneration was observed 2 days after infection, whereas no cell degeneration was observed in the MDCK cells expressing EGFP. These results indicate that the binding of EGFP to DegNb8 enhanced the replication ability of DegNb8-NP-containing IAV, and decreased the replication ability of NP-DegNb8-containing IAV. Next, the growth characteristics of DegNb8-NP- or NP-DegNb8-containing IAV were compared with those of wild-type IAV using the HA assay (Fig. 10(d)). In the MDCK cells expressing EGFP, the differences in peak HA titers between wild-type IAV and DegNb8-NP- or NP-DegNb8-containing IAV were 2²-fold and 2⁶-fold, respectively. On the other hand, in the MDCK cells not expressing EGFP, the differences were 2⁵-fold and 2⁴-fold, respectively. These results revealed that DegNb8-NP-containing IAV can replicate efficiently in cells containing EGFP, whereas NP-DegNb8-containing IAV can replicate efficiently in cells not containing EGFP.

### Example 7

### Generation of Influenza Virus Containing Two Types of Nanobodies and Infection

The pcDNA3-PolI-PB1-LaM4 prepared in Example 2 and the pcDNA3-PolI-PB2-DegNb7 prepared in Example 5 were used to generate influenza virus containing two types of nanobodies. The HEK293T and MDCK cells were seeded one day prior to transfection on one 3.5 cm culture dish with 2.5 x 10⁵ cells/2 ml each. 0.35 µg of each of the eight pcDNA-PolI plasmids and 1 µg of pcDNA3-EGFP plasmid for EGFP expression were transfected using Lipofectamine LTX and Opti-MEM. At 24 hpt, the cells were washed twice with phosphate-buffered saline (PBS), the medium was then replaced with DMEM containing 0.2% bovine serum albumin (BSA, Sigma-Aldrich), 100 units/mL penicillin, 100 mg/mL streptomycin, and 1 mg/mL N-p-Tosyl-L-phenylalanine chloromethyl ketone (TPCK)-treated trypsin (Sigma-Aldrich), and the cells were cultured at 37°C in 0.5% CO₂ for 2 days to produce IAV containing two types of nanobodies, PB1-LaM4 and PB2-DegNb7. MDCK cells expressing neither EGFP nor mCherry, MDCK cells expressing EGFP, MDCK cells expressing mCherry, and MDCK cells expressing both EGFP and mCherry were infected with wild-type IAV or IAV containing two types of nanobodies, PB1-LaM4 and PB2-DegNb7, at an MOI of 1 in an infection medium (DMEM containing 0.2% BSA, 100 units/mL penicillin, 100 mg/mL streptomycin, and 0.5 mg/mL TPCK-treated trypsin). At 48 hours post-infection, the cells were washed with PBS and stained with 1% crystal violet. In addition, MDCK cells expressing neither EGFP nor mCherry, MDCK cells expressing EGFP, MDCK cells expressing mCherry, and MDCK cells expressing both EGFP and mCherry were infected with wild-type IAV or IAV containing two types of nanobodies, PB1-LaM4 and PB2-DegNb7, at an MOI of 0.1, and the growth characteristics over 3 days were measured using the HA assay.

### Generation of Replication-Competent IAV Containing Two Types of Nanobodies

In the MDCK cells expressing EGFP, cell degeneration (apoptosis) due to the replication of IAV containing two types of nanobodies, PB1-LaM4 and PB2-DegNb7, was observed 2 days after infection (Fig. 11(c)). On the other hand, only slight cell degeneration was observed in the MDCK cells expressing neither EGFP nor mCherry, and in the MDCK cells expressing mCherry. No cell degeneration was observed in the MDCK cells expressing mCherry. Next, the growth characteristics of IAV containing two types of nanobodies, PB1-LaM4 and PB2-DegNb7, were compared with those of wild-type IAV using the HA assay (Fig. 11(d)). In the MDCK cells expressing EGFP, the difference in peak HA titers between wild-type IAV and IAV containing two types of nanobodies, PB1-LaM4 and PB2-DegNb7, was 2²-fold. On the other hand, in the MDCK cells expressing neither EGFP nor mCherry, MDCK cells expressing mCherry, and MDCK cells expressing both EGFP and mCherry, the differences were 2⁵-fold, 2⁶-fold, and 2⁵-fold, respectively. These results revealed that IAV containing two types of nanobodies, PB1-LaM4 and PB2-DegNb7, can replicate efficiently in cells containing EGFP. It was also revealed that replication was inhibited in cells that contained EGFP but also mCherry. This indicates that combining two types of nanobodies can more precisely restrict the cells in which IAV replicates.

### References

Reference 1: Hoffmann E, Neumann G, Kawaoka Y, Hobom G, Webster RG. A DNA transfection system for generation of influenza A virus from eight plasmids. PNAS 2000; 97:6108-6113.

Reference 2: Manicassamy B, Manicassamy S, Belicha-Villanueva A, Pisanelli G, Pulendran B, Garcia-Sastre A. Analysis of in vivo dynamics of influenza virus infection in mice using a GFP reporter virus. PNAS 2010; 107: 11531-11536.

Reference 3: Schmidt TGM, Eichinger A, Schneider M, Bonet L, Carl U, Karthaus D, Theobald I, Skerra A. The role of changing loop conformations in streptavidin versions engineered for high-affinity binding of the strep-tag II peptide. J Mol Biol 2021; 433: 166893.

Reference 4: Bethuyne J, de Gieter S, Zwaenepoel O, Garcis-Pino A, Durinck K, Verhelle A, Hassanzadeh-Ghassabeh G, Speleman F, Loris R, Gettemans J. A nanobody madulates the p53 transcriptional program without perturbing its functional architecture. Nucleic Acids Res 2014; 42: 12928-12938.

Reference 5: Fridy PC, Li Y, Keegan S, Thompson MK, Nudelman I, Scheid JF, Oeffinger M, Nussenzweig MC, Fenyo D, Chait BT, Rout MP. A robust pipeline for rapid production of versatile nanobady repertoires. Nat Methods 2014; 11: 1253-1260.

### Industrial Applicability

The present invention can be used in research, medical, and other fields.

Sequence Listing

## Claims

1. A method for controlling vRNA transcription and/or replication by influenza virus RNA-dependent RNA polymerase (RdRp), comprising forming at least one selected from the group consisting of subunits constituting the RdRp and nucleoprotein (NP) into a fusion protein with another protein.

2. The method according to claim 1, wherein at least one of the subunits constituting the RdRp is a fusion protein with another protein.

3. The method according to claim 1, wherein the other protein interacts with a target substance.

4. The method according to claim 3, wherein the state of vRNA transcription and/or replication by RdRp changes between a state in which the other protein and the target substance interact and a state in which they do not interact.

5. Modified RdRp in which at least one of subunits constituting influenza virus RdRp is constructed as a fusion protein with another protein, and the other protein interacts with a target substance.

6. The RdRp according to claim 5, wherein the other protein is any one selected from the group consisting of 1) a protein with specific binding affinity to a target substance, 2) a light-responsive protein, 3) an ion-responsive protein, 4) a small molecule-binding protein, 5) a nucleic acid-binding protein, and 6) a temperature-responsive protein.

7. The RdRp according to claim 5 or 6, wherein the subunit is constructed as a fusion protein with the other protein at the C-terminus of the subunit.

8. The RdRp according to claim 5 or 6, wherein the other protein is 100 kDa or less in size.

9. A nucleic acid construct that expresses the subunit constructed as a fusion protein with another protein and contained in the modified RdRp of claim 5, or that expresses a modified viral gene corresponding to the subunit.

10. The nucleic acid construct according to claim 9, which is a nucleic acid construct for controlling vRNA transcription and/or replication by RdRp.

11. A nucleic acid construct for controlling vRNA transcription and/or replication by RdRp, which expresses NP constructed as a fusion protein with another protein and contained in modified NP, in which NP of influenza virus is constructed as a fusion protein with another protein, and the other protein interacts with a target substance, or which expresses a modified viral gene corresponding to the NP.

12. A composition for producing modified influenza virus, comprising the nucleic acid construct of any one of claims 9 to 11.

13. The composition according to claim 12, for use in vectors or cell lysis.
